# EUROPEAN PATENT APPLICATION

(11) **EP 3 449 918 A1**
(43) Date of publication of application: **06.03.2019**
(21) Application number: 17189054.4
(22) Date of filing: 01.09.2017
(51) Int. Cl.: A61K 31/14, A61K 31/155, A61K 31/352, A61K 31/404, A61K 31/445, A61K 31/454, A61K 31/495, A61K 31/519, A61K 31/5415, A61K 31/553, A61P 19/10

(54) **BMP-MIMETICS**

(71) Applicant: Technische Universität Dortmund, 44227 Dortmund (DE); Ernst-Moritz-Arndt-Universität Greifswald, 17489 Greifswald (DE)
(72) Inventor: SCHADE, Dennis, 17489 Greifswald (DE); HALVER, Jonas, 44227 Dortmund (DE)
(74) Representative: Viering, Jentschura & Partner mbB Patent- und Rechtsanwälte

(57) **Abstract**

The present invention relates to a method for differentiating, transdifferentiating or reprogramming a cell by contacting a cell with a compound as defined in the present invention. The invention further relates to said compounds as defined in the present invention for use in the treatment of osteoporosis, bone fracture, osteonecrosis, osteoarthritis, rheumatoid arthritis, spondylosis, anterior lumbar fusion or long bone fracture and to a method for determining bone morphogenetic protein (BMP) mimetic activity of a compound of interest.

## Description

### FIELD OF THE INVENTION

The present invention lies in the field of medicinal chemistry and chemical biology and relates to a method for differentiating, transdifferentiating or reprogramming a cell by contacting a cell with a compound as defined in the present invention. The invention further relates to said compounds as defined in the present invention for use in the treatment of osteoporosis, bone fracture, osteonecrosis, osteoarthritis, rheumatoid arthritis, spondylosis, anterior lumbar fusion or long bone fracture and to a method for determining bone morphogenetic protein (BMP) mimetic activity of a compound of interest.

### BACKGROUND OF THE INVENTION

Bone morphogenetic proteins (BMPs) are cytokines with diverse functions belonging to the ligands of the TGFβ superfamily. They were originally named after their ability to promote the formation of bones *in vivo* (Chen et al., 2004, Reddi et al., 2009). Based on their sequence homology, BMPs are divided into four groups: BMP2/4, BMP5/6/7 /8a /8b, BMP9/10, and BMP12/13/14 (Bragdon et al., 201 1, Begam et al., 2017). Their influence on the differentiation of stem cells is not limited to the stimulation of osteogenesis, but is also found in the development and homeostasis of a large number of human organ systems (Wagner et al., 2010, Lowery et al., 2016) and their activity is associated with several pathologies (Salazar et al., 2016, Morrell et al., 2016, Lowery et al., 2010).

After BMPs are bound to the transmembrane BMP receptor type I, this receptor recruits the type II receptor that is thereby activated. The active type I receptor possesses an intracellular serine/threonine kinase that phosphorylates the intracellular effectors Smad1 and Smad5, which leads to their separation from the receptor complex. The phosphorylated Smad1 and Smad5 then form a complex with Smad4 and translocate into the nucleus to activate the transcription of their target genes (Derynck et al., 1998, Massague et al., 2000). The BMP/Smad pathway is also negatively regulated by structurally and functionally different Smad effector proteins of the subfamily "inhibitory Smads" (= Smad 6 and Smad 7). The Smad effector proteins are subsequently degraded, whereby the ubiquitin-ligase Smurf1 plays a decisive role.

While there is a plurality of inhibitors of the BMP signal transduction cascade, only a few activators are known. Primarily the activation of the signal pathway in the absence of BMP ligands is a great challenge.

BMP mimetic compounds may have a huge potential, particularly as drugs with therapeutic purposes and as molecules that can be used in biotechnological applications.

Osteopenic diseases such as osteoporosis, bone fractures, inflammation-related bone destruction, osteoarthritis or rheumatoid arthritis are an enormous challenge for the health and economic system as well as society. Compounds having BMP mimetic activity are a promising tool for the treatment of the above-mentioned diseases.

As concerns the use of BMP mimetic compounds in biotechnology, the BMP signaling pathway is a major regulator of developmental biology, various BMPs are important for the use of stem cell-based technologies. They can be used specifically to influence the fate of stem and precursor cells. On the one hand, this is the so-called "directed differentiation" of embryonic and/or inductive pluripotent stem cells into desired specialized target cells and tissues. In this context, BMPs are used in the induction of mesoderm and are thus used for the generation of all cell/tissue types which are derived from the mesoderm: a) cardiovascular precursor cells and myoblasts, which later form the cells of the blood vessels, the heart muscle and the skeletal muscle; b) hematopoietic stem cells, which form all cells of the blood; c) mesenchymal stem cells (MSCs), which are the starting point for cells of adipose tissue, bone and cartilage.

The use of BMP4, for example, is particularly well-suited for the targeted generation of cardiac precursor cells and myocardial cells from human stem cells (Burridge et al., 2014, Zhang et al., 2015, Rao et al., 2016).

In addition to the directed differentiation from pluripotent stem cells, BMPs (especially BMP4) can be used in the so-called "transdifferentiation" of fibroblasts to form cardiac muscle cells. Such methods have been described in the art (Wang et al 2014, Cao et al 2016, Zhang et al., 2016). The transdifferentiation is a process in which not necessarily requires a pluripotent stem cell, but rather terminally differentiated cells (such as fibroblasts) are intermediately transformed into a multipotent state (de-differentiation), in order to subsequently differentiate into the desired target cell.

Hence, there is need in the art for improved methods for differentiating, transdifferentiating or reprogramming cells, methods for determining bone morphogenetic protein (BMP) mimetic activity of a compound of interest and the treatment of osteoporosis, bone fracture, osteonecrosis, osteoarthritis, rheumatoid arthritis, spondylosis, anterior lumbar fusion or long bone fracture.

### SUMMARY OF THE INVENTION

It is an object of the present invention to meet the above need by providing methods for differentiating, transdifferentiating or reprogramming a cell by contacting said cell with a compound as defined in the present invention. Further the present invention provides compounds as defined herein for use in the treatment of osteoporosis, bone fracture, osteonecrosis, osteoarthritis, rheumatoid arthritis, spondylosis, anterior lumbar fusion or long bone fracture and a method for determining bone morphogenetic protein (BMP) mimetic activity of a compound of interest. Surprisingly, the present inventors have found a narrow time frame during differentiation of murine embryonic stem cells to cardiac muscle cells in which BMP activity has a cardiomyogenesis blocking effect. When BMP activity is blocked during said specific time frame (for example by selective BMP inhibitors, such as DMH1), the tested cells show expression of cardiac muscle cell specific genes after at least eight days of treatment. This expression (or the lack of expression) can be used as readout for BMP activity. Compounds that should be tested for BMP mimetic activity are applied to the cells in parallel with the BMP inhibitor. When no expression of cardiac muscle cell specific genes is observed after all steps of the differentiation protocol have been applied to the cells, the compound is believed to exhibit cardiomyogenesis blocking and BMP mimetic activity.

By using the above-described screening assay, the present inventors identified ten compounds that have BMP mimetic activity. Such compounds can be used for biotechnological applications, such as differentiation, transdifferentiation or reprogramming applications, or for the treatment of diseases, such as osteoporosis, bone fracture, osteonecrosis, osteoarthritis, rheumatoid arthritis, spondylosis, anterior lumbar fusion or long bone fracture.

In a first aspect, the present invention relates to a method for differentiating, transdifferentiating or reprogramming a cell, said method comprising:
contacting said cell with at least one compound selected from the group consisting of

   I) A-B-C,

   wherein A, B and C are independently from each other substituted or unsubstituted 4-6-membered heterocarbocycles,
   said compound comprises in total 2-6 nitrogen atoms,
   said compound comprises in total at least three, preferably at least four double bonds,
   wherein the heterocarbocycles A and B share two atoms, and
   wherein the heterocarbocycles B and C share two atoms;

   II) A-B,

   wherein A and B are independently from each other substituted or unsubstituted 4-6-membered heterocarbocycles,
   said compound comprises in total 2-6 nitrogen atoms,
   said compound comprises in total at least two, preferably at least three double bonds, and
   wherein the heterocarbocycles A and B share two atoms;

   III) B-C,

   wherein B and C are independently from each other substituted or unsubstituted 4-6-membered heterocarbocycles,
   said compound comprises in total 2-6 nitrogen atoms,
   said compound comprises in total at least two, preferably at least three double bonds, and
   wherein the heterocarbocycles B and C share two atoms; 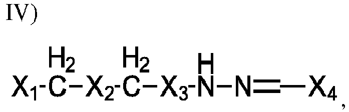
   wherein
   X₁ is a substituted or unsubstituted 5-6-membered aromatic (hetero)cyclyl,
   X₂ is a linear substituted or unsubstituted heteroalkyl having up to 10 atoms comprising at least one nitrogen atom or a substituted or unsubstituted heterocyclyl having 4-10 atoms comprising at least one nitrogen atom;
   X₃ is selected from the group consisting of C=O, S=O, O=S=O, and 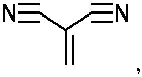 and
   X₄ is a substituted or unsubstituted 4-6-membered aromatic (hetero)cyclyl; 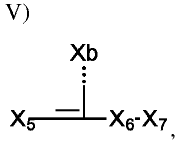
   wherein
   X₅ is selected from the group consisting of 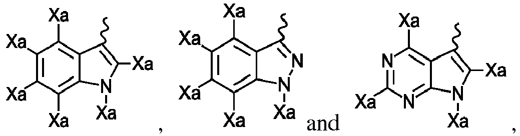 wherein Xa is H, halogen or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms,
   X₆ is selected from the group consisting of C=O, S=O, O=S=O, and 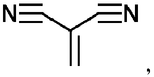 and
   X₇ is a substituted or unsubstituted 5-6-membered aromatic (hetero)cyclyl,
   X_{b} is selected from the group consisting of N, NO₂, COOH, COX, COX_{H}, and CONH₂, wherein
   X is H or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms,
   X_{H} is selected from the group consisting of F, Cl, Br and I,
   ------ is a) a triple bond if X_{b} is N or b) a single bond if X_{b} is NO₂, COOH, COX, COX_{H}, or CONH₂;

   VI) **Yₐ-X₈-X₉-Yₐ,**

   wherein
   X₈ is a linear substituted or unsubstituted alkyl having up to 10 carbon atoms,
   X₉ is a substituted or unsubstituted 4-6-membered (hetero)cyclyl, and
   Ya is 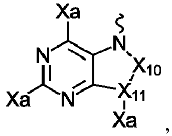 wherein
   Xa is as defined above,
   X₁₀ is selected from the group consisting of C=O, S=O, O=S=O, 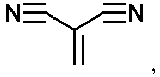 and a carbon atom linked to two moieties selected from H, halogen, a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, and substituted or unsubstituted heteroalkylaryl, each having up to 20 carbon atoms, and
   X₁₁ is NH, S or O; 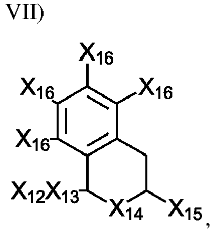
   wherein
   X₁₂ is NH₂, OH or SH,
   X₁₃ is a linear substituted or unsubstituted alkyl having up to 10 carbon atoms,
   X₁₄ is O, S or NH,
   X₁₅ is a substituted or unsubstituted (hetero)cyclyl having up to 20 carbon atoms, and
   X₁₆ is H or OH; 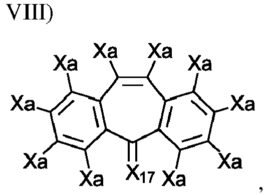
   wherein
   Xa is as defined above, and
   X₁₇ is a substituted or unsubstituted 4-6-membered (hetero)cyclyl; 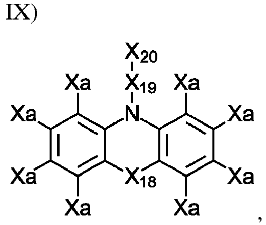
   wherein
   Xa is as defined above,
   X₁₈ is S, O or NH,
   X₁₉ is a linear substituted or unsubstituted alkyl having up to 10 carbon atoms, and
   X₂₀ is a substituted or unsubstituted 4-6-membered (hetero)cyclyl; 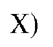 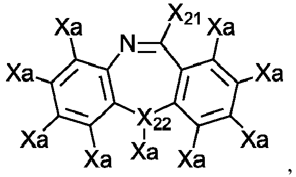
   wherein
   Xa is as defined above,
   X₂₁ is a substituted or unsubstituted 4-6-membered (hetero)cyclyl, and
   X₂₂ is S, O or NH; 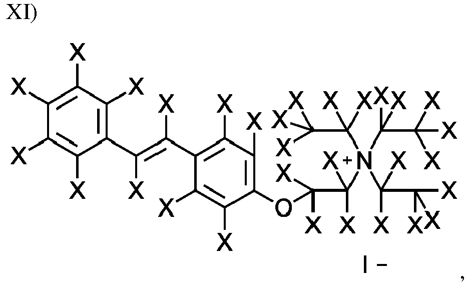
   wherein
   X is H or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms; and I⁻ is an anion; or 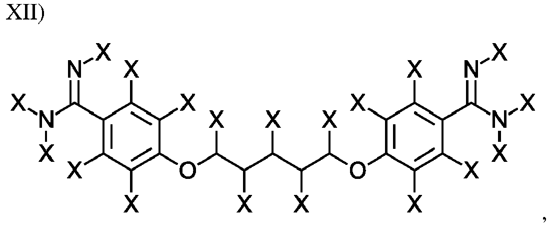 wherein
   X is as defined above,
   to differentiate, transdifferentiate or reprogram said cell.

In vatious embodiments of the above method,
a) in A-B-C
   i) A is a substituted or unsubstituted 6-membered heterocarbocycle;
   ii) B is a substituted or unsubstituted 6-membered heterocarbocycle;
   iii) C is a substituted or unsubstituted 5-membered heterocarbocycle;
   iv) the number nitrogen atoms is four;
   v) A comprises 1-4 halogen atoms;
   vi) B comprises at least one nitrogen atom;
   vii) C comprises a (hetero)aryl substituent; and/or
   viii) the ring fusion between B and C is in an angle between 115°-125°; or
b) in A-B
   i) A is a substituted or unsubstituted 6-membered heterocarbocycle;
   ii) B is a substituted or unsubstituted 6-membered heterocarbocycle;
   iii) the number nitrogen atoms is two;
   iv) A comprises 1-4 halogen atoms; and/or
   v) B comprises at least one nitrogen atom;
c) in B-C
   i) B is a substituted or unsubstituted 6-membered heterocarbocycle;
   ii) C is a substituted or unsubstituted 5-membered heterocarbocycle;
   iii) the number nitrogen atoms is four;
   v) B comprises at least one nitrogen atom; and/or
   vi) C comprises a (hetero)aryl substituent.

In still further various embodiments,
A-B-C, A-B or B-C are selected from the group consisting of 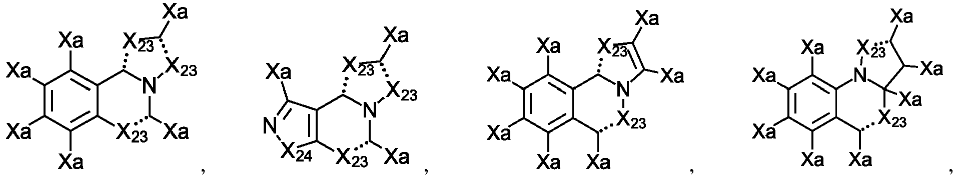 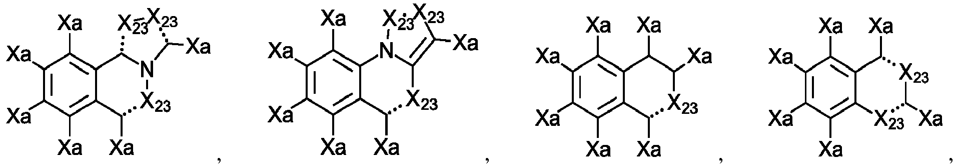 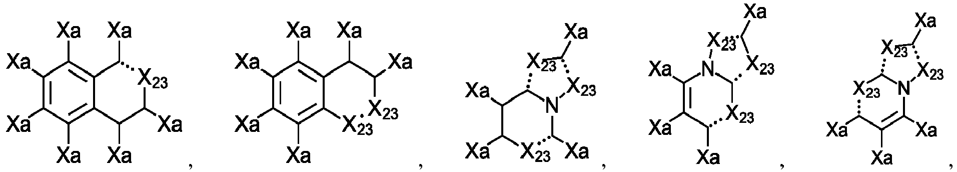 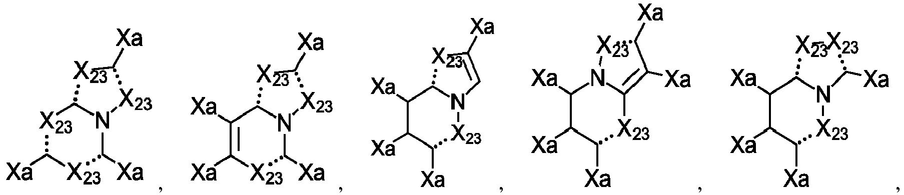 and 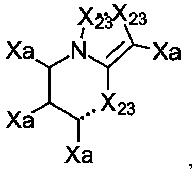 wherein
X₂₃ is N, O, S, or N-Xa,
X₂₄ is N, O, or S,
Xa is as defined above, and
------ is a) a single bond if X₂₃ is O, S, or N-Xa or b) a double bond if X₂₃ is N.

In various embodiments, the compound is selected from the group consisting of 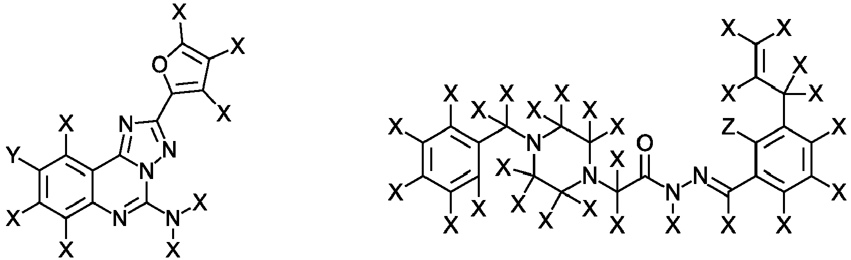 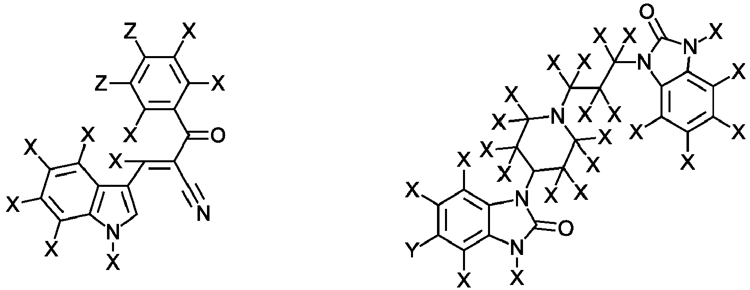 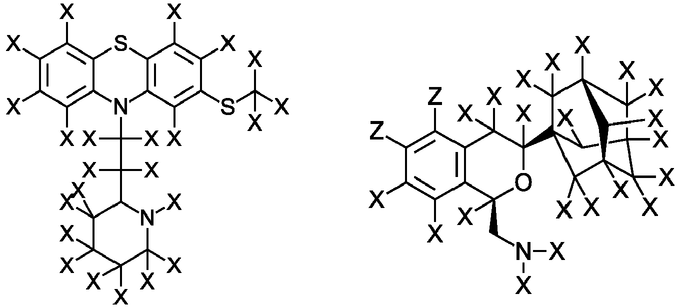 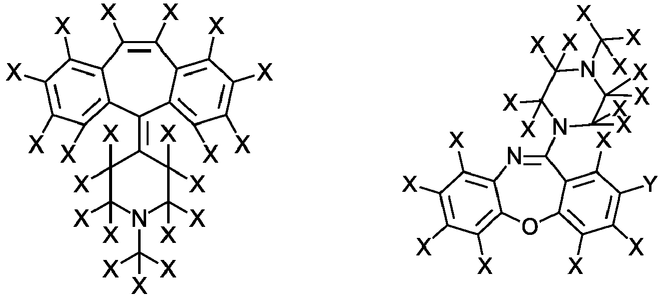 wherein
X is H or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms;
Y is halogen; and
Z is a linear or branched, substituted or unsubstituted hydroxyl group having up to 10 carbon atoms.

In various embodiments, X is H.

In various embodiments of the invention, the at least one compound is selected from the group consisting of 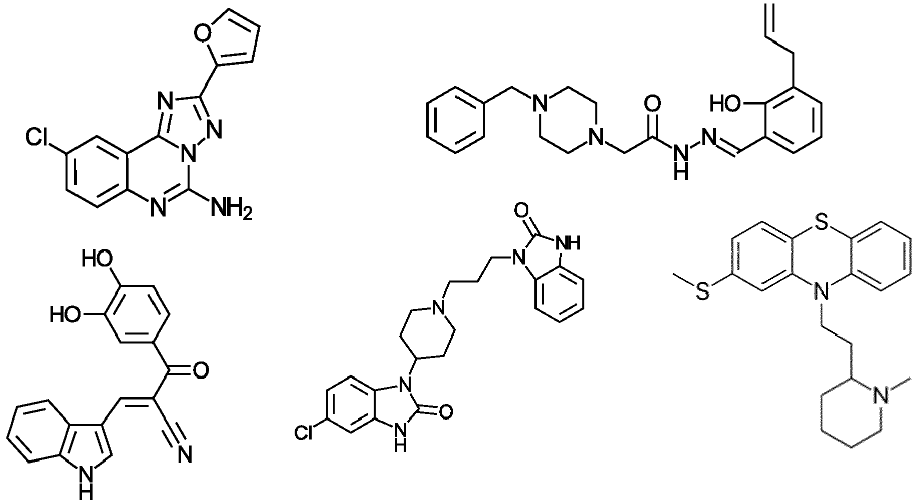 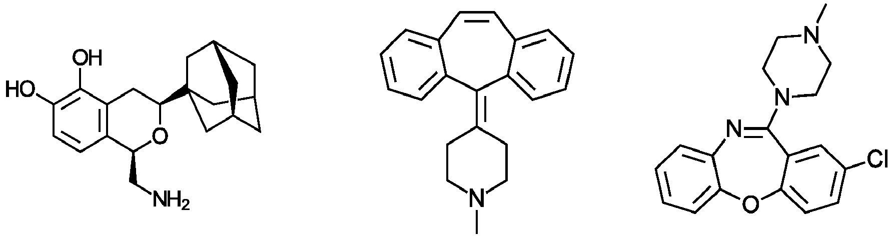 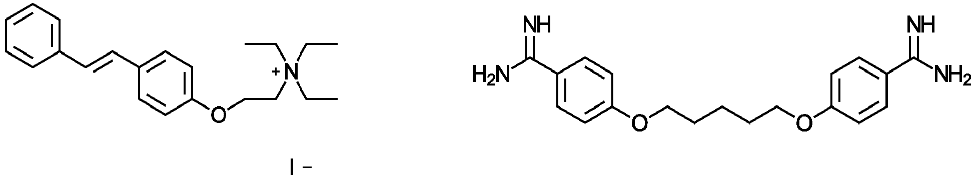
(I) said method is a method to differentiate a cell and
   (a) the cell is a pluripotent stem cell; and/or
   (b) the cell differentiates into a multipotent cell, preferably a cardiac progenitor cell, myoblast, hematopoietic stem cell or mesenchymal stem cell; and/or
   (c) the differentiation further comprises contacting the cell with at least one compound supporting differentiation;
(II) said method is a method to transdifferentiate a cell and
   (a) the cell is a fibroblast; and/or
   (b) the cell transdifferentiates into a cardiac muscle cell; and/or
   (c) the transdifferentiation further comprises contacting the cell with at least one compound supporting transdifferentiation; or
(III) said method is a method to reprogram a cell and
   (a) the cell is a fibroblast; and/or
   (b) the cell reprograms into a pluripotent stem cell; and/or
   (c) the reprogramming further comprises contacting the cell with at least one compound selected from the group of Oct-4, Sox-2, Klf-4 or c-Myc.

In a second aspect, the present invention is directed to a compound as described above for use in the treatment of osteoporosis, bone fracture, osteonecrosis, osteoarthritis, rheumatoid arthritis, spondylosis, anterior lumbar fusion or long bone fracture.

In a third aspect, the present invention relates to a method for determining bone morphogenetic protein (BMP) mimetic activity of a compound of interest, said method comprising:
(a) culturing a pluripotent stem cell, wherein said stem cell comprises an inducible mesoderm or cardiac reporter molecule;
(b) after at least one day of step (a) and for a time frame of up to five days, contacting the cell with the compound of interest and with a BMP inhibitor, preferably said BMP inhibitor is selected from the group consisting of 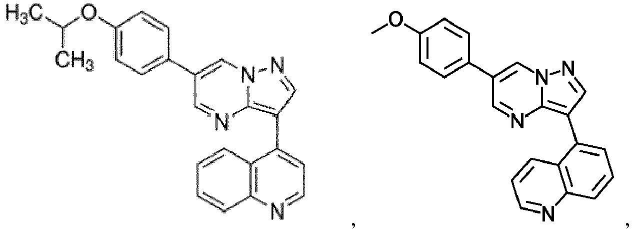 Noggin (NOG) and Chordin;
(c) after the time frame of step (b), replacing the medium of the cultured stem cell;
(d) after at least nine days of step (a), comparing the presence of the reporter molecule in the stem cell with the presence of the same reporter molecule in a control cell, wherein in step (b) the control cell is (I) only contacted with the BMP inhibitor; or (II) contacted with the BMP inhibitor and a compound having BMP mimetic activity; and
(e)
   (I) wherein the control cell has only been contacted with the BMP inhibitor and the compound of interest has BMP mimetic activity when the presence of the reporter molecule in the stem cell is significantly reduced compared to the presence of the reporter molecule in the control cell; or
   (II) wherein the control cell has been contacted with the BMP inhibitor and a compound having BMP mimetic activity and the compound of interest has BMP mimetic activity when the presence of the reporter molecule in the stem cell does not significantly differ compared to the presence of the reporter molecule in the control cell.

In various embodiments, the inducible mesoderm or cardiac reporter molecule is a nucleic acid molecule comprising a promoter of a cardiac muscle indicator gene and a reporter molecule, wherein the promoter and the reporter molecule are operatively coupled.

In various embodiments, the compound having BMP mimetic activity and applied to the control cell is a compound selected from the group consisting of

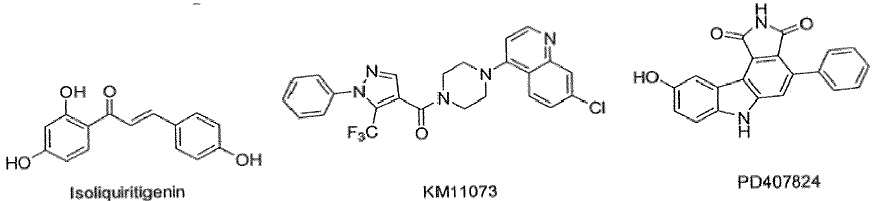

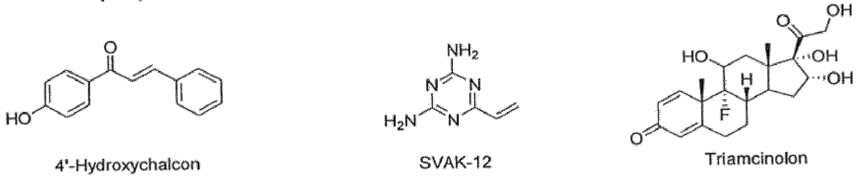

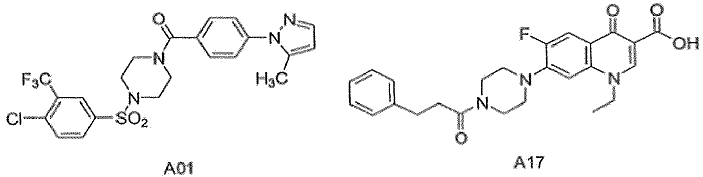

In various embodiments, the pluripotent stem cells is cultured in step (a) and/or in step (c) with differentiation medium that does not comprise the leukemia inhibitory factor (LIF).

In various embodiments, the method is directed to methods wherein (a) the pluripotent stem cell is a murine embryonic stem cell, preferably a CGR8 cell; and/or (b) said method comprises an additional step of replacing the medium eight days after the culturing step (a); and/or (c) the compound of interest is further tested for BMP mimetic activity using a reporter construct comprising a BMP-responsive element.

In various embodiments of the present invention, the promoter of the cardiac muscle indicator gene is the promoter of one of the following genes: NPPB (natriuretic peptide B), MYL7 (myosin, light chain 7), NPPA (natriuretic peptide A), MYBPC3 (myosin binding protein C, cardiac), TNNT2 (troponin T type 2 (cardiac)), BMP10 (bone morphogenetic protein 10), TNNI3 (troponin I type 3 (cardiac)), MYH6 (myosin, heavy chain 6, cardiac muscle, alpha), ANKRD1 (ankyrin repeat domain 1 (cardiac muscle)), RD3L (retinal degeneration 3-like), SBK2 (SH3 domain binding kinase family, member 2), and MYL4 (myosin, light chain 4, alkali; atrial, embryonic), Nkx2.5, Mef2c, Isl-1, cKit, Sea1, preferably MYH6.

In various embodiments, the reporter molecule is a fluorescence reporter molecule, preferably green fluorescent protein (GFP).

In various embodiments, the contacting step (b) is done at least two days, preferably three days after the culturing step (a) and/or the time frame of the contacting step (b) is three days, preferably one day.

In various embodiments of the present invention, the comparing step (d) is done eleven days after the culturing step (a).

It is understood that all combinations of the above disclosed embodiments are also intended to fall within the scope of the present invention.

### BRIEF DESCRIPTION OF THE DRAWINGS

The invention will be better understood with reference to the detailed description when considered in conjunction with the non-limiting examples and the accompanying drawings.

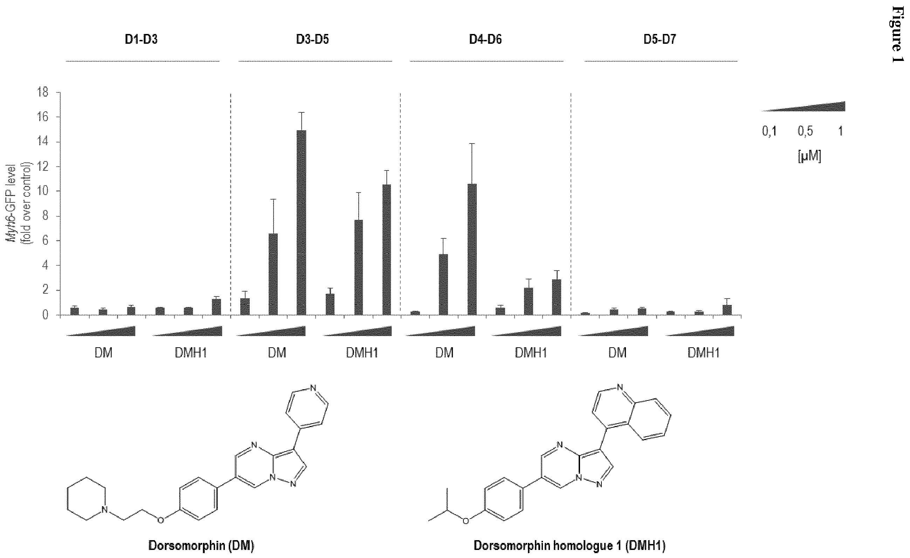

**Figure 1** shows data to establish an assay system in murine embryonic stem cells (mESCs) to identify bone morphogenetic protein (BMP) mimetic compounds. The BMP inhibitors dorsomorphine (DM) and DMH1 demonstrate depending on their individual selective inhibitory profile different pro-cardiac activity.

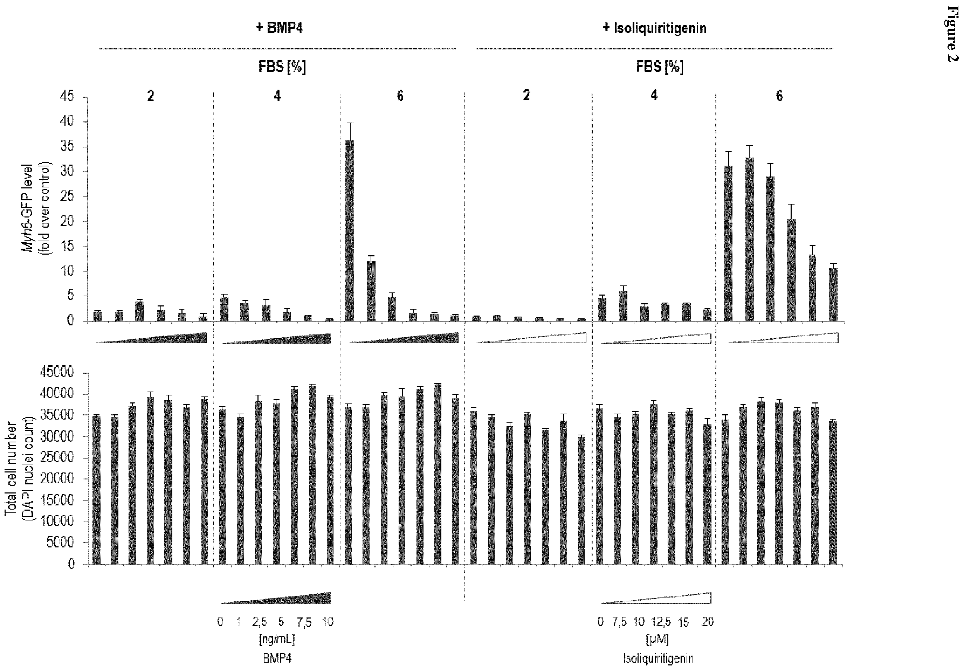

**Figure 2** shows that the DMH1 induced cardiomyogenesis, which is induced between days 3 and 4, can be completely antagonized by BMP4, whereas isoliquirtigenin only demonstrates an incomplete inhibition of the described effect.

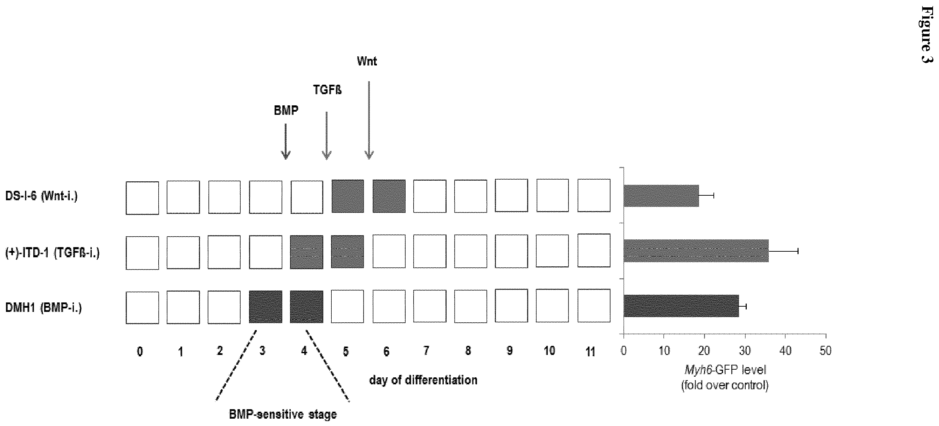

**Figure 3** shows a summary of the pro-cardiac effects of BMP inhibition (DMH1), TGFβ inhibition (DHP-1) and Wnt/β-catenin inhibition (IWR type inhibitor DS-I-6).

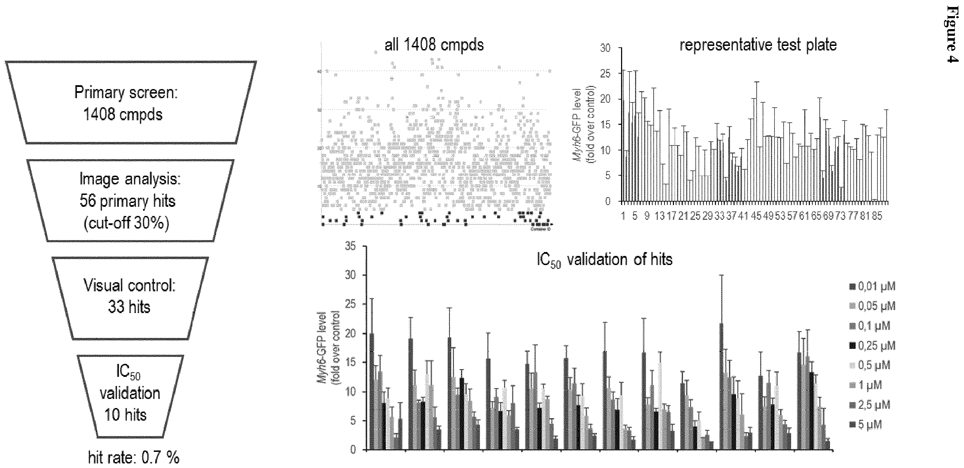

**Figure 4** shows that a high content-screening of the LOPAC compound library using the screening assay of the invention identifies BMP mimetic compounds. A workflow is provided including an overview of the whole screen (1408 compounds) and a representative screening result of a 384 well plate testing 90 compounds. For 33 hits the IC₅₀ value was determined, wherein 10 compounds demonstrates a dosage dependent BMP mimetic activity.

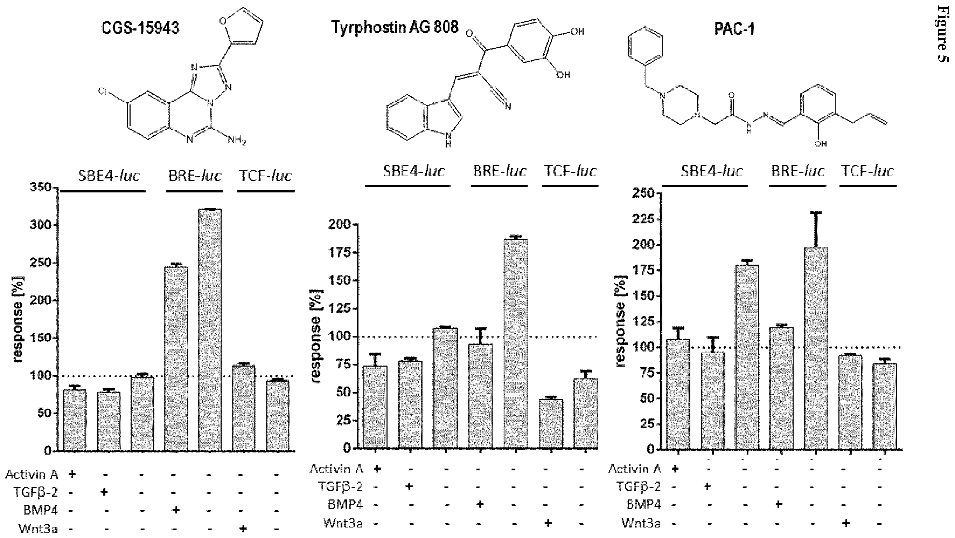

**Figure 5** shows the signal pathway selectivity of three of the ten compounds identified as hits in the screening assay of the invention. HEK 293T cells are transiently transfected with the following reporter constructs: SBE4-luc (Smad4-binding element luciferase reporter construct), BRE-luc (BMP-responsive element luciferase reporter construct), TCF-luc (TCF/LEF transcriptional response element). The signal pathways were stimulated with 10 ng/mL of the corresponding growth factor and the hit compounds were tested at a concentration of 5 µM.

### DETAILED DESCRIPTION OF THE INVENTION

The present inventors surprisingly found a time frame during differentiation of stem cells in which BMP activity alone can block the further differentiation of the stem cell (cardiomyogenesis blocking activity). This time frame can be used to screen for compounds with BMP mimetic activity. Based on their observation, the present inventors have established a high content screening assay to screen for BMP mimetics. By using their screening assay and a compound library of 1408 compounds, the present inventors found ten compounds that exhibit BMP mimetic activity.

Thus, in a first aspect, the present invention relates to a method for differentiating, transdifferentiating or reprogramming a cell, said method comprising:
contacting said cell with at least one compound selected from the group consisting of

   I) A-B-C,

   wherein A, B and C are independently from each other substituted or unsubstituted 4-6-membered heterocarbocycles,
   said compound comprises in total 2-6 nitrogen atoms,
   said compound comprises in total at least three, preferably at least four double bonds,
   wherein the heterocarbocycles A and B share two atoms, and
   wherein the heterocarbocycles B and C share two atoms;

   II) A-B,

   wherein A and B are independently from each other substituted or unsubstituted 4-6-membered heterocarbocycles,
   said compound comprises in total 2-6 nitrogen atoms,
   said compound comprises in total at least two, preferably at least three double bonds, and
   wherein the heterocarbocycles A and B share two atoms;

   III) B-C,

   wherein B and C are independently from each other substituted or unsubstituted 4-6-membered heterocarbocycles,
   said compound comprises in total 2-6 nitrogen atoms,
   said compound comprises in total at least two, preferably at least three double bonds, and
   wherein the heterocarbocycles B and C share two atoms; 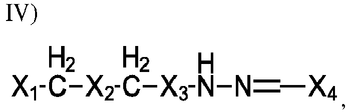
   wherein
   X₁ is a substituted or unsubstituted 5-6-membered aromatic (hetero)cyclyl,
   X₂ is a linear substituted or unsubstituted heteroalkyl having up to 10 atoms comprising at least one nitrogen atom or a substituted or unsubstituted heterocyclyl having 4-10 atoms comprising at least one nitrogen atom;
   X₃ is selected from the group consisting of C=O, S=O, O=S=O, and 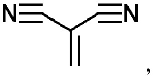 and
   X₄ is a substituted or unsubstituted 4-6-membered aromatic (hetero)cyclyl;

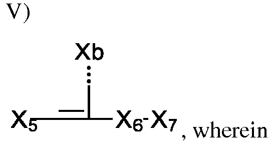

   X₅ is selected from the group consisting of 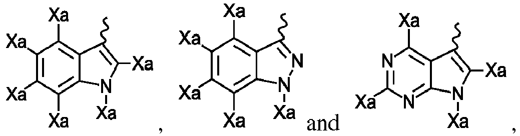 wherein Xa is H, halogen or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms,
   X₆ is selected from the group consisting of C=O, S=O, O=S=O, and 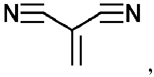 and
   X₇ is a substituted or unsubstituted 5-6-membered aromatic (hetero)cyclyl,
   X_{b} is selected from the group consisting of N, NO₂, COOH, COX, COX_{H}, and CONH₂, wherein
   X is H or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms,
   X_{H} is selected from the group consisting of F, Cl, Br and I,
   ------ is a) a triple bond if X_{b} is N or b) a single bond if X_{b} is NO₂, COOH, COX, COX_{H}, or CONH₂;

   VI) **Yₐ-X₈-X₉-Yₐ,**

   wherein
   X₈ is a linear substituted or unsubstituted alkyl having up to 10 carbon atoms,
   X₉ is a substituted or unsubstituted 4-6-membered (hetero)cyclyl, and
   Ya is 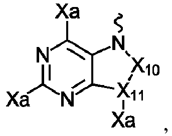 wherein Xa is as defined above,
   X₁₀ is selected from the group consisting of C=O, S=O, O=S=O, 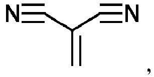 and a carbon atom linked to two moieties selected from H, halogen, a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, and substituted or unsubstituted heteroalkylaryl, each having up to 20 carbon atoms, and
   X₁₁ is NH, S or O; 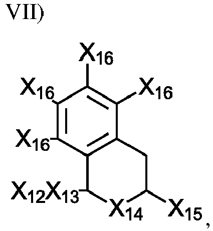
   wherein
   X₁₂ is NH₂, OH or SH,
   X₁₃ is a linear substituted or unsubstituted alkyl having up to 10 carbon atoms,
   X₁₄ is O, S or NH,
   X₁₅ is a substituted or unsubstituted (hetero)cyclyl having up to 20 carbon atoms, and
   X₁₆ is H or OH; 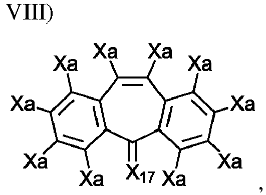
   wherein
   Xa is as defined above, and
   X₁₇ is a substituted or unsubstituted 4-6-membered (hetero)cyclyl; 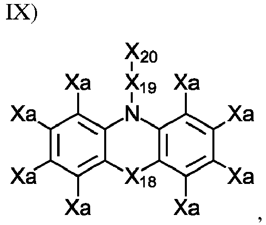
   wherein
   Xa is as defined above,
   X₁₈ is S, O or NH,
   X₁₉ is a linear substituted or unsubstituted alkyl having up to 10 carbon atoms, and
   X₂₀ is a substituted or unsubstituted 4-6-membered (hetero)cyclyl; 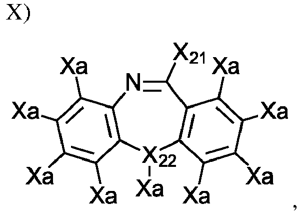
   wherein
   Xa is as defined above,
   X₂₁ is a substituted or unsubstituted 4-6-membered (hetero)cyclyl, and
   X₂₂ is S, O or NH; 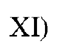 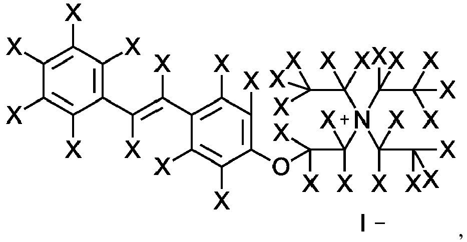
   wherein
   X is H or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms; and I⁻ is an anion; or 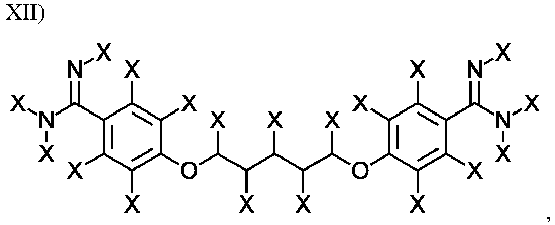 wherein
   X is as defined above,
   to differentiate, transdifferentiate or reprogram said cell.

In vatious embodiments of the above method,
a) in A-B-C
   i) A is a substituted or unsubstituted 6-membered heterocarbocycle;
   ii) B is a substituted or unsubstituted 6-membered heterocarbocycle;
   iii) C is a substituted or unsubstituted 5-membered heterocarbocycle;
   iv) the number nitrogen atoms is four;
   v) A comprises 1-4 halogen atoms;
   vi) B comprises at least one nitrogen atom;
   vii) C comprises a (hetero)aryl substituent; and/or
   viii) the ring fusion between B and C is in an angle between 115°-125°; or
b) in A-B
   i) A is a substituted or unsubstituted 6-membered heterocarbocycle;
   ii) B is a substituted or unsubstituted 6-membered heterocarbocycle;
   iii) the number nitrogen atoms is two;
   iv) A comprises 1-4 halogen atoms; and/or
   v) B comprises at least one nitrogen atom;
c) in B-C
   i) B is a substituted or unsubstituted 6-membered heterocarbocycle;
   ii) C is a substituted or unsubstituted 5-membered heterocarbocycle;
   iii) the number nitrogen atoms is four;
   v) B comprises at least one nitrogen atom; and/or
   vi) C comprises a (hetero)aryl substituent.

The term "ring fusion angle", as used herein, refers to an angle which is formed by two lines, namely L_{A} and L_{B}. L_{A} refers to a line which is perpendicular to a between the ring atoms commonly shared by A and B. L_{B} refers to a line which is perpendicular to a between the ring atoms commonly shared by B and C. The ring fusion angle is between 115°-125°. In various embodiments, the ring fusion angle is between 116°-124°, 117°-123°, 118°-122°, 119°-121° or 119.5°-120.5°. In preferred embodiments, the ring fusion angle is 120°.

In still further various embodiments,
A-B-C, A-B or B-C are selected from the group consisting of 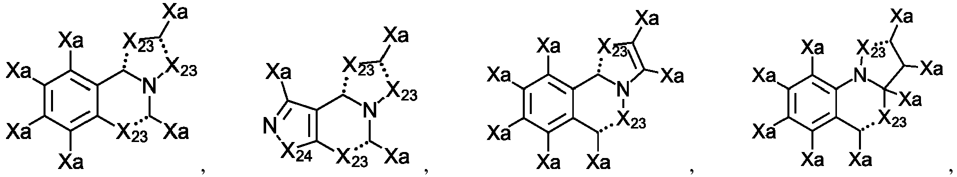 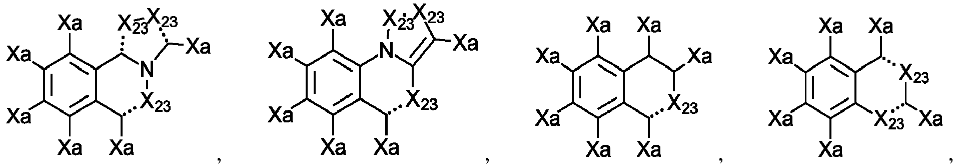 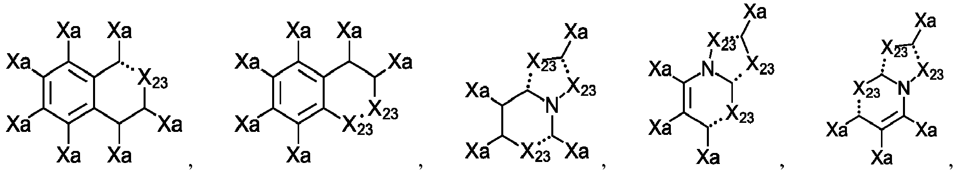 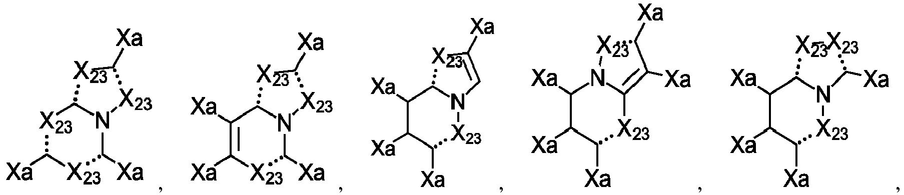 and 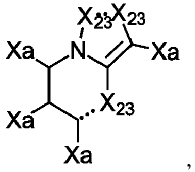 wherein
X₂₃ is N, O, S, or N-Xa,
X₂₄ is N, O, or S,
Xa is as defined above, and
------ is a) a single bond if X₂₃ is O, S, or N-Xa or b) a double bond if X₂₃ is N.

In various embodiments, the compound is selected from the group consisting of 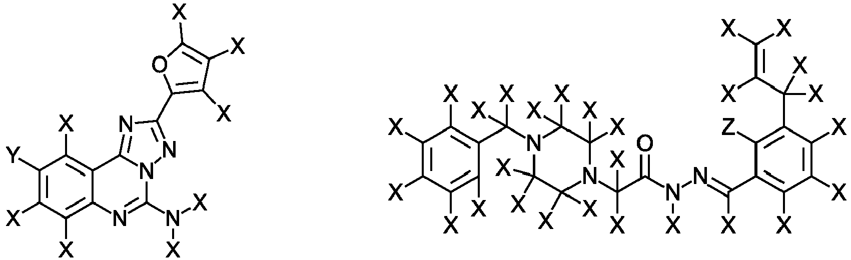 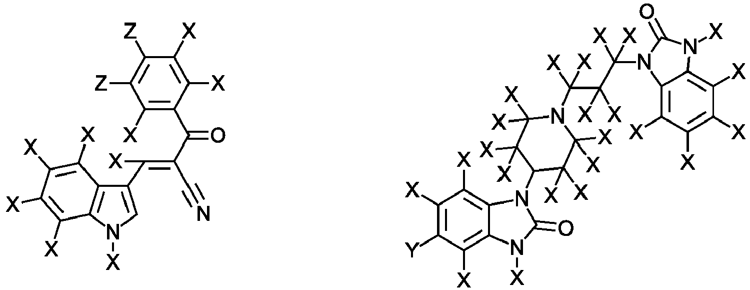 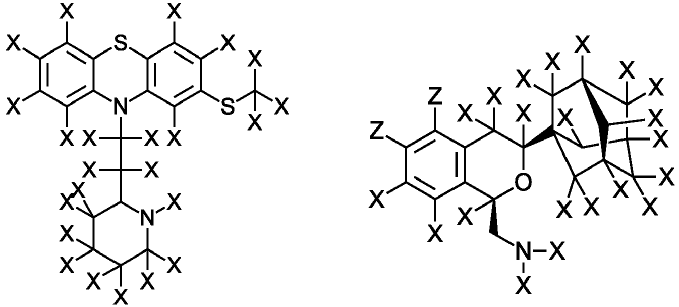 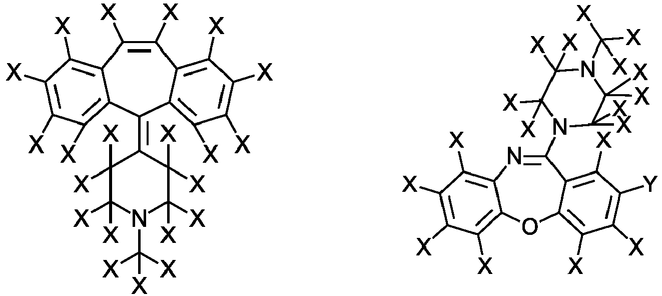 wherein
X is H or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms;
Y is halogen; and
Z is a linear or branched, substituted or unsubstituted hydroxyl group having up to 10 carbon atoms.

The terms "differentiation" or "direct differentiation", as interchangeably used herein, refers to a process of converting pluripotent or multipotent cell to a desired terminally differentiated cell (for example a somatic cell). For example, differentiation refers to a process by which precursor or progenitor cells differentiate into specific cell types such as osteocytes, cardiac cells and chondrocytes. The term "inducing differentiation", as used herein, is taken to mean causing a stem cell to develop into a specific differentiated cell type as a result of a direct or intentional influence on the stem cell. Influencing factors can include cellular parameters such as ion influx, a pH change and/or extracellular factors, such as secreted proteins or small molecule compounds (such as the compounds described herein), growth factors and cytokines that regulate and trigger differentiation. It may include culturing the cell to confluence and may be influenced by cell density.

The term "transdifferentiation", as used herein, refers to the process that starts with de-differentiation of terminally differentiated cell (somatic cell A, unipotent) to a multipotent state, followed by differentiation to a different terminally differentiated cell type (somatic cell B). Said process does not comprise a pluripotent state. Thus, transdifferentiation describes the conversion of a non-stem cell into a different type of cell. Transdifferentiation is a type of metaplasia, which is the replacement of one differentiated cell type with another differentiated cell type. It is generally caused by some sort of abnormal stimulus such as a dramatic change in the cell's environment. Cell types are distinct morphological or functional forms of cells.

The terms "reprogramming" and "reprogram", as used herein, refer to the dedifferentiation of terminally differentiated cells (such as somatic cells) to a) pluripotent cells or b) multipotent cells (= part of transdifferentiation). Thus, reprogramming is a process involving the re-differentiation or de-differentiation of a cell having the morphological and functional characteristics representative of a certain class of cells, into a cell having known morphological and functional characteristics of a different class of cells. Thus, for example, fibroblasts may be reprogrammed to pluripotent stem cells.

The term "dedifferentiate" or "dedifferentiation", as used herein, refers to the process by which lineage committed cells (e.g. myoblasts or osteoblasts) reverse their lineage commitment and become precursor or progenitor cells (i.e. multipotent or pluripotent stem cells).

"Redifferentiation", as used herein, refers to a process by which a group of once differentiated cells return to their original specialized form.

The above terms are defined in more detail in Xie, M. et al. (Xie, M. et al. (2017) Acc Chem Res.;50(5):1202-1211), which is herewith incorporated by reference.

The term "cell", as used herein, can refer to a single and/or isolated cell or to a cell that is part of a multicellular entity such as a tissue, an organism or a cell culture. In other words the methods can be performed *in vivo, ex vivo* or *in vitro.* The cell is a eukaryotic cell. A eukaryotic cell, as used herein, refers to any cell of a multi-cellular eukaryotic organism, including cells from animals like vertebrates. Preferably, the cell is a mammalian cell. The term "mammalian cell", as used herein, is well known in the art and refers to any cell belonging to or derived from an animal that is grouped into the class of mammalia. Cells of different mammalian subclasses such as prototheria or theria may be used. For example, within the subclass of theria, preferably cells of animals of the infraclass eutheria, more preferably of the order primates, artiodactyla, perissodactyla, rodentia and lagomorpha are used in the methods of the invention. Furthermore, within a species one may choose a cell to be used in the methods of the invention based on the tissue type and/or capacity to differentiate equally depending on the goal to be achieved by modifying the genome via transducing a target cell according to the method of the invention. There are three basic categories of cells that make up the mammalian body: germ cells, somatic cells and stem cells. A germ cell is a cell that gives rise to gametes and thus is continuous through the generations. Stem cells can divide and differentiate into diverse specialized cell types as well as self-renew to produce more stem cells. In mammals there are two main types of stem cells: embryonic stem cells and adult stem cells. Somatic cells include all cells that are not a gametes, gametocytes or undifferentiated stem cells. The cells of a mammal can also be grouped by their ability to differentiate. A totipotent (also known as omnipotent) cell is a cell that is able to differentiate into all cell types of an adult organism including placental tissue such as a zygote (fertilized oocyte) and subsequent blastomeres, whereas pluripotent cells, such as embryonic stem cells, cannot contribute to extraembryonic tissue such as the placenta, but have the potential to differentiate into any of the three germ layers endoderm, mesoderm and ectoderm. Multipotent progenitor cells have the potential to give rise to cells from multiple, but limited number of cell lineages. Further, there are oligopotent cells that can develop into only a few cell types and unipotent cells (also sometimes termed a precursor cell) that can develop into only one cell type. There are four basic types of tissues: muscle tissue, nervous tissue, connective tissue and epithelial tissue that a cell can be derived from, such as for example hematopoietic stem cells or neuronal stem cells. To the extent human cells are envisaged for use in the methods of the invention, it is preferred that such human cell is not obtained from a human embryo, in particular not via methods entailing destruction of a human embryo. On the other hand, human embryonic stem cells are at the skilled person's disposal such as taken from existent embryonic stem cell lines commercially available. Accordingly, the present invention may be worked with human embryonic stem cells without any need to use or destroy a human embryo. Alternatively, or instead of human embryonic stem cells, pluripotent cells that resemble embryonic stem cells such induced pluripotent stem (iPS) cells may be used, the generation of which is state of the art (Hargus G et al., 2010, Proc Natl Acad Sci U S A, 107:15921-15926; Jaenisch R. and Young R., 2008, Cell 132:567-582; Saha K, and Jaenisch R., 2009, Cell Stem Cell 5:584-595).

The term "alkyl," by itself or as part of another substituent, means, unless otherwise stated, a straight (i.e. unbranched) or branched chain, or combination thereof, which is be fully saturated and can include di- and multivalent radicals, having the number of carbon atoms designated (i.e. C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like.

The term "alkylene" by itself or as part of another substituent means a divalent radical derived from an alkyl, as exemplified, but not limited, by -CH₂CH₂CH₂CH₂-. Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The term "heteroalkyl," by itself or in combination with another term, means, unless otherwise stated, a stable straight or branched chain, or cyclic hydrocarbon radical, or combinations thereof, consisting of at least one carbon atoms and at least one heteroatom selected from the group consisting of O, N, P, Si and S, and wherein the nitrogen and sulfur atoms may optionally be oxidized and the nitrogen heteroatom may optionally be quaternized. The heteroatom(s) O, N, P and S and Si may be placed at any interior position of the heteroalkyl group or at the position at which the alkyl group is attached to the remainder of the molecule. Examples include, but are not limited to, -CH₂-CH₂-O-CH₃,-CH₂-CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, O-CH₃, -O-CH₂-CH₃, and -CN. Up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-. As described above, heteroalkyl groups, as used herein, include those groups that are attached to the remainder of the molecule through a heteroatom, such as -C(O)R', -C(O)NR', -NR'R", -OR', -SR', and/or -SO₂R'. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as-NR'R" or the like.

The terms "cycloalkyl", "heterocycloalkyl" and "(hetero)cyclyl", by themselves or in combination with other terms, represent, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like. A "cycloalkylene" and "heterocycloalkylene" refer to a divalent radical derived from cycloalkyl and heterocycloalkyl, respectively.

The terms "halo" or "halogen," by themselves or as part of another substituent, mean, unless otherwise stated, a fluorine, chlorine, bromine, or iodine atom. Preferably, the halogen is chlorine.

The term "aryl" means, unless otherwise stated, a polyunsaturated, aromatic, hydrocarbon substituent which can be a single ring or multiple rings (preferably from 1 to 3 rings) which are fused together or linked covalently. The term "heteroaryl" refers to aryl groups (or rings) that contain from one to four heteroatoms selected from N, O, and S, wherein the nitrogen and sulfur atoms are optionally oxidized, and the nitrogen atom(s) are optionally quaternized. A heteroaryl group can be attached to the remainder of the molecule through a carbon or heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below. "Arylene" and "heteroarylene" refers to a divalent radical derived from a aryl and heteroaryl, respectively.

The term "arylalkyl" is meant to include those radicals in which an aryl group is attached to an alkyl group (e.g., benzyl, phenethyl, pyridylmethyl and the like). The term "heteroarylalkyl" includes the above described groups wherein one or more carbon atoms of the alkyl or aryl portion (e.g., a methylene group) are replaced by, for example, an oxygen atom (e.g., phenoxymethyl, 2-pyridyloxymethyl, 3-(1-naphthyloxy)propyl, and the like).

Where a heteroalkyl, heterocycloalkyl, or heteroaryl includes a specific number of members (e.g. "3 to 7 membered"), the term "member" refers to a carbon or heteroatom.

The term "oxo" as used herein means an oxygen that is double bonded to a carbon atom.

Each of the above terms (e.g., "alkyl," "heteroalkyl," "aryl" and "heteroaryl") are meant to include both substituted and unsubstituted forms of the indicated radical. Preferred substituents for each type of radical are provided below.

Substituents for the alkyl, heteroalkyl, alkylene, alkenyl, heteroalkylene, heteroalkenyl, alkynyl, cycloalkyl, heterocycloalkyl, cycloalkenyl, and heterocycloalkenyl radicals can be one or more of a variety of groups selected from, but not limited to: -OR', =O, =NR', =N-OR', -NR'R", -SR',-halogen, -SiR'R"R''', -OC(O)R', -C(O)R', -CO₂R', -CONR'R", -OC(O)NR'R",-NR"C(O)R', -NR'-C(O)NR"R''', -NR"C(O)₂R', -NR-C(NR'R"R''')=NR'''', -NR-C(NR'R")=NR''', -S(O)R', -S(O)₂R', -S(O)₂NR'R", -NRSO₂R', -CN and -NO₂ in a number ranging from zero to (2 m'+1), where m' is the total number of carbon atoms in such radical. R', R", R''' and R'''' each preferably independently refer to hydrogen, substituted or unsubstituted heteroalkyl, substituted or unsubstituted cycloalkyl, substituted or unsubstituted heterocycloalkyl, substituted or unsubstituted aryl (e.g., aryl substituted with 1-3 halogens), substituted or unsubstituted alkyl, alkoxy or thioalkoxy groups, or arylalkyl groups. When a compound of the invention includes more than one R group, for example, each of the R groups is independently selected as are each R', R", R'" and R'''' groups when more than one of these groups is present. When R' and R" are attached to the same nitrogen atom, they can be combined with the nitrogen atom to form a 4-, 5-, 6-, or 7-membered ring. For example, -NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (e.g., -CF₃ and -CH₂CF₃) and acyl (e.g., -C(O)CH₃, -C(O)CF₃, -C(O)CH₂OCH₃, and the like).

Similar to the substituents described for the alkyl radical, substituents for the aryl and heteroaryl groups are varied and are selected from, for example: halogen, -OR', -NR'R", -SR', -halogen,-SiR'R"R''', -OC(O)R', -C(O)R', -CO₂R', -CONR'R", -OC(O)NR'R", -NR"C(O)R', -NR'-C(O)NR"R'", -NR"C(O)₂R', -NR-C(NR'R"R''')=NR'''', -NR-C(NR'R")=NR''', -S(O)R',-S(O)₂R', -S(O)₂NR'R", -NRSO₂R', -CN and -NO₂, -R', -N₃, -CH(Ph)₂, fluoro(C₁-C₄)alkoxy, and fluoro(C₁-C₄)alkyl, in a number ranging from zero to the total number of open valences on the aromatic ring system; and where R', R", R''' and R'''' are preferably independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. When a compound of the invention includes more than one R group, for example, each of the R groups is independently selected as are each R', R", R'" and R'''' groups when more than one of these groups is present.

Further, in preferred embodiments, two substituents on adjacent ring atoms can be combined to form a cyclic group that may be saturated, unsaturated or aromatic and selected from aryl, heteroaryl, cycloalkyl, cycloheteroalkyl, all of which may be substituted as defined for the respective groups above.

As used herein, the term "heteroatom" or "ring heteroatom" is meant to include oxygen (O), nitrogen (N), sulfur (S), phosphorus (P), and silicon (Si).

The term "alkenyl" as used herein is a hydrocarbon group of from 2 to 20 carbon atoms with a structural formula containing at least one carbon-carbon double bond. Asymmetric structures such as (A1A2)C=C(A3A4) are intended to include both the E and Z isomers. The alkenyl group can be substituted with one or more groups including, but not limited to, alkyl, cycloalkyl, alkoxy, alkenyl, cycloalkenyl, alkynyl, aryl, heteroaryl, aldehyde, amino, carboxylic acid, ester, ether, halide, hydroxy, ketone, azide, nitro, silyl, sulfo-oxo, or thiol, as described herein.

The term "cycloalkenyl" as used herein is a non-aromatic carbon-based ring composed of at least three carbon atoms and containing at least one carbon-carbon double bound, i.e., C=C. Examples of cycloalkenyl groups include, but are not limited to, cyclopropenyl, cyclobutenyl, cyclopentenyl, cyclopentadienyl, cyclohexenyl, cyclohexadienyl, norbornenyl, and the like. The cycloalkenyl group can be substituted or unsubstituted. The cycloalkenyl group can be substituted with one or more groups including, but not limited to, alkyl, cycloalkyl, alkoxy, alkenyl, cycloalkenyl, alkynyl, aryl, heteroaryl, aldehyde, amino, carboxylic acid, ester, ether, halide, hydroxy, ketone, azide, nitro, silyl, sulfo-oxo, or thiol as described herein.

The term "alkynyl" as used herein is a hydrocarbon group of 2 to 20 carbon atoms with a structural formula containing at least one carbon-carbon triple bond. The alkynyl group can be unsubstituted or substituted with one or more groups including, but not limited to, alkyl, cycloalkyl, alkoxy, alkenyl, cycloalkenyl, alkynyl, aryl, heteroaryl, aldehyde, amino, carboxylic acid, ester, ether, halide, hydroxy, ketone, azide, nitro, silyl, sulfo-oxo, or thiol, as described herein.

Substituents R, R₁ and/or R₂, if present in any one of the substituents described herein, replace the hydrogen atom(s) of the respective ring atom.

The term "hydroxyl group", as used herein, is meant to include the -OH groups such as are contained in alcohols, phenols, and carboxylic acids. The preferred types of hydroxyl groups are linear alcohols. In other preferred embodiments, the linear alcohols comprise one, two or three -OH groups (more preferably one -OH group) and comprise 1-10, 2-8, 3-6 carbon atoms.

In various embodiments, X is H.

In various embodiments of the invention, the at least one compound is selected from the group consisting of

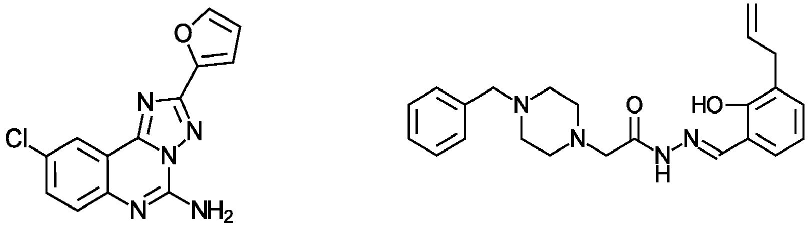

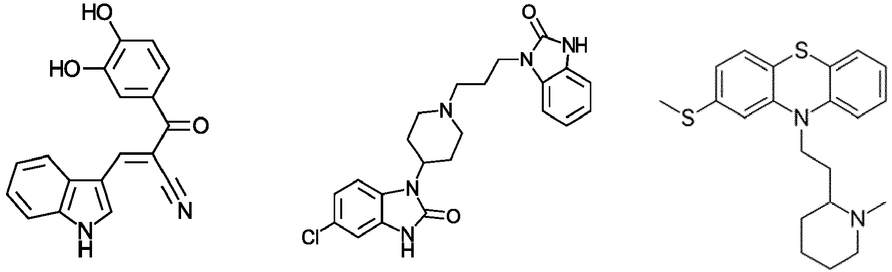

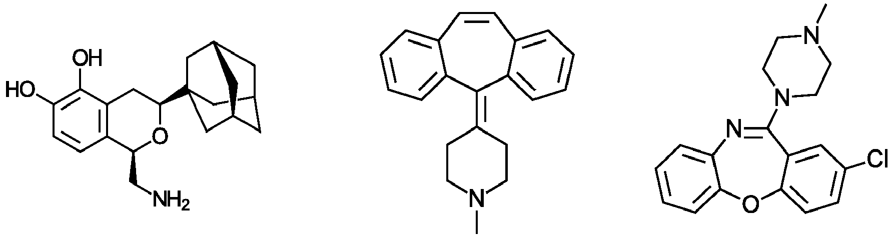

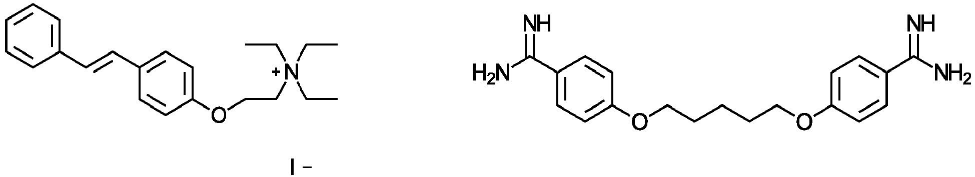

In various embodiments, said method is a method to differentiate a cell and wherein (a) the cell is a pluripotent stem cell; and/or (b) the cell differentiates into a cardiac progenitor cell, myoblast, hematopoietic stem cell or mesenchymal stem cell; and/or (c) the differentiation further comprises contacting the cell with at least one compound supporting differentiation.

Methods to differentiate cells involving the use of BMP activity are well-known in the art. For example, the use of BMP4 to generate cardiac progenitor cells or cardiac muscle cells from human stem cells is disclosed in Burridge et al. 2014, Zhang et al. 2015 and Rao et al. 2016 whose content is herewith incorporated by reference. In addition, BMPs and BMP mimetic compounds can be used specifically to influence the fate of stem and precursor cells. This "directed differentiation" of embryonic and/or inductive pluripotent stem cells into desired specialized target cells and tissues can involve BMPs or BMP mimetic compounds which are used for the induction of mesoderm. In the art there well-known protocols available to differentiate stem and progenitor cells to: a) cardiovascular precursor cells and myoblasts, which later form the cells of the blood vessels, the heart muscle and the skeletal muscle; b) hematopoietic stem cells, which form all cells of the blood; c) mesenchymal stem cells (MSCs), which are the starting point for cells of adipose tissue, bone and cartilage.

In various embodiments, the method to differentiate a cell comprises converting a cell, preferably a stem cell, into a mesodermal cell, preferably a cardiac progenitor, cardiomyocyte, or hemangioblast. In alternative embodiments, the method to differentiate a cell comprises converting a cell, preferably a stem cell, into an ectodermal cell, preferably a epithelial stem cell. In other alternative embodiments, the method to differentiate a cell comprises converting a cell, preferably a stem cell, into an endodermal cell, preferably a hepatocyte progenitor or immature lung cell.

Stem cells are cells in multicellular organisms that can divide and differentiate into diverse specialized cell types and can self-renew to produce more stem cells that have the same property. A "pluripotent stem cell", as used herein, is a stem cell that has the potential to differentiate into any of the three germ layers: endoderm (e.g., interior stomach lining, gastrointestinal tract, the lungs), mesoderm (e.g., muscle, bone, blood, urogenital system), and ectoderm (e.g., epidermal tissues and nervous system). Pluripotent stem cells can give rise to any fetal or adult cell type. For the purposes of this disclosure a "pluripotent stem cell" may include a totipotent stem cell, which is a cell that can construct a complete, viable organism. These cells are produced from the fusion of an egg and sperm cell. Cells produced by the first few divisions of the fertilized egg are also totipotent. Pluripotent stem cells include but are not limited to embryonic stem (ES) cells, induced pluripotent stem cells (iPSC), and cells produced by somatic cell nuclear transfer (SCNT).

ES cells are totipotent stem cells derived from the inner cell mass of the blastocyst of an early-stage mammalian embryo. Methods of deriving mammalian ES cells (including whose that are not destroying an embryo) are well known in the art as are numerous established ES cell lines that may be used in conjunction with certain embodiments of this disclosure.

The term "(human) cardiac progenitor cell", as used herein, refers to a (human) progenitor cell that is committed to the cardiac lineage and that has the capacity to differentiate into all three cardiac lineage cells (cardiac muscle cells, endothelial cells and smooth muscle cells). The term "(human) cardiomyogenic progenitor cell", as used herein, refers to a human progenitor cell that is committed to the cardiac lineage and that predominantly differentiates into cardiac muscle cells (i.e., more than 10% of the differentiated cells, preferably more than 20%, 30%, 40%, 50%, 60%, 70%, 80% or 90% of the differentiated cells, derived from the progenitor cells are cardiac muscle cells). The term "cardiomyocyte" refers to a muscle cell of the heart (e.g. a cardiac muscle cell). A cardiomyocyte will generally express on its cell surface and/or in the cytoplasm one or more cardiac-specific marker. Suitable cardiomyocyte-specific markers include, but are not limited to, cardiac troponin I, cardiac troponin-C, tropomyosin, caveolin-3, GATA-4, myosin heavy chain, myosin light chain-2a, myosin light chain-2v, ryanodine receptor, and atrial natriuretic factor.

The term "myoblast", as used herein, refers to an embryonic cell in the mesoderm that differentiates to give rise to a muscle cell or myocyte. The term includes cells *in vivo* and cells cultured *ex vivo* regardless of whether such cells are primary or passaged.

"Hematopoietic stem cell", as used herein, refers to a cell, e.g., a bone marrow cell, or a fetal liver or spleen cell, which is capable of developing into all myeloid and lymphoid lineages and by virtue of being able to self-renew can provide long term hematopoietic reconstitution. Purified preparations of hematopoietic cells or preparations, such as bone marrow, which include other cell types, can be used in methods of the invention. Although not wishing to be bound by theory, it is believed that the hematopoietic stem cells home to a site in the recipient. Hematopoietic stem cells can be from fetal, neonatal, immature or mature animals. Stem cells derived from the cord blood of the recipient or the donor can be used in methods of the invention (see U.S. Patent 5,192,553, hereby incorporated by reference, and U.S. Patent 5,004,681, hereby incorporated by reference).

The phrase "mesenchymal stem cell", as used herein, refers to a stem cell of mesenchymal origin. Mesenchymal stem cells are multipotent stromal cells that can differentiate into a variety of cell types, including osteoblasts, chondrocytes and adipocytes.

In alternative embodiments, said method is a method to transdifferentiate a cell and wherein (a) the cell is a fibroblast; and/or (b) the cell transdifferentiates into a cardiac muscle cell; and/or (c) the transdifferentiation further comprises contacting the cell with at least one compound supporting transdifferentiation.

In various embodiments, compounds supporting differentiation and/or transdifferentiation comprise signaling pathway modulators, epigenetic modulators, small molecules with diverse modes of action and growth factors.

In preferred embodiments, signaling pathway modulators comprise
1) Wnt/β-Catenin stimulators and GSK3 inhibitors, such as CHIR99021, BIO, kenpaullone, LiCl, Wnt ligands (e.g. Wnt-3a);
2) Wnt/β-Catenin inhibitors, such as
   - Tankyrase inhibitors (such as XAV939, NVP-TNKS656, Compound 25 and analogs, IWR-1, 53AH and analogs),
   - Porcupine inhibitors (such as IWP-2, IWP-4, Wnt-C59, LGK974 and analogs),
   - β-Catenin protein-protein-inhibitors (such as iCRT-14 and analogs, ICG-001, IQ-1, StAx-35R and analogs, TU-002, windorphen, carnosic acid),
   - diverse compounds (such as cardionogen-1, KY-02111, pyrvinium, SB-203580, TA-02, CCT251545 and anlogs, CCT031374), and
   - Dickkopf-1 (Dkk1)
3) modulators of non-canonical Wnt signaling (such as Wnt ligands/proteins),
4) TGFβ/Activin/Nodal stimulators (such as TGFβ isoforms, Activin A, Nodal),
5) TGFβ/Activin inhibitors (such as A83-01, SB431542, RepSox, LY-2157299, galunisertib, LY-2109761, GW788388, SB-525334, SB-505124, EW-7203 and analogs, (+)-*R*-ITD-1, ITD-ts and analogs),
6) Sonic hedgehog (Shh) signaling modulators, such as
   - stimulators: such as SAG, Smoothened, Hh-Ag1.5, purmorphamine, SHH (= ligand), and
   - inhibitors: such as Shh inhibitors (LDE225, cyclopamine, robotnikinin),
7) retinoic acid signaling modulators (such as retinoic acid, BMS-189453),
8) miscellaneous signaling pathway modulators, such as
   - PDGF, VEGF, FGF inhibitors (such as JNJ10198409, SU16F, SU5402, PD-173074),
   - VEGF signaling mimetics, such as ONO-1301, FGF signaling mimetics such as SUN-11602,
   - G-CSF mimetic: such as SB-247464,
   - EGFR inhibitors (such as erlotinib),
   - Notch signaling inhibitors (such as Compound E),
   - Hippo/Yap signaling modulators (such as cardiac glycoside digitoxin, C108, verteporfin, protoporphyrin IX, 9E, MST1 and MST2 inhibitors such as XMU-MP-1),
   - P38-MAPK inhibitors (such as SB-203580, SB-23906, skepinon-L etc.),
   - MAP/ERK/MEK inhibitors (such as PD0325901, SU1498, SC1 = Pluripotin, PD-184352),
   - PI3K inhibitors (such as LY294002),
   - ROCK inhibitors (such as Y27632, thiazovivin),
   - Raf inhibitors (such as ZM336372),
   - JAK inhibitors (such as JI1),
   - JNK inhibitor (such as SP600125),
   - IGF agonists (such as IGF-1) and IGF antagonists,
   - CaMKII inhibitors (such as KN62, KN93),
   - mTOR inhibitors (such as Rapamycin).

In preferred embodiments, epigenetic modulators comprise
- AMI-5 (such as PRMT inhibitors, HLMT inhibitors),
- AS8351 (such as histone demethylase inhibitors, KDM5B),
- RG108, 5-Aza, 5-Aza-dc, 3-Deazaneplanocin A (DNA methyltransferase inhibitor),
- BIX-01294, EPZ004777, SGC0946 (G9a inhibitor and histone methyltransferase inhibitor),
- sodium butyrate, valproic acid, Suberanilohydroxamic acid (SAHA), Trichostatin A (HDAC inhibitors),
- Parnate (LSD1inhibitor, KDM inhibitor, histone methyltransferase inhibitor),
- pVc (2-phosphorylated vitamin C).

In preferred embodiments, miscellaneous small molecules with different and diverse modes of action comprise Cardiogenol C (and analogs), VUT-MK142, Shz-1, Icariin, CW209E, CZ-27, 8Br-cADPR, triiodo-L-thyronin (T3), 1-EBIO, AG1478, Lithium chloride, Ascorbic acid, Isx-1, Isx2 (and derivatives), Forskolin (Adenynyl cyclase inhibitor, cAMP activator), OAC2 (Oct4 enhancer), Lysophosphatidic acid (LPA), PS48, AlCAR, SMER28, R428 (Axl inhibitor), Ca-channel inhibitors (nifedipine, verapamil), BAY-K8644, PGE2, dm-PGE2, PGD2, AM1241, AhR antagonist (SRI), UM171, P7C3, Metformin, AMD3100 (plerixafor), BIO5192, Sphingosin-1-phosphate (SIP), CASIN, indolactam V (ILV), IDE-2, stauprimide, SB-497115.

In preferred embodiments, growth factors comprise
- Bone Morphogenetic Protein (BMP) isoforms (BMP-2, -3, -4, -5, -6, -7, -8, -9, -12, -13, -14),
- Thrombopoetin (TPO), erythropoetin (EPO), Colony-stimulating factors (such as G-CSF), interleukins (such as IL2, IL6, IL11), stem cell factor (SCF)/cKit-Ligand, HGF, VEGF, insulin, PDGF, thymosin β4, IGF1, neuregulin-1 (NRG1), FGFs (FGF-2, -3, -10, -11, -13,-15), LIF, SHH, Wnt ligands, TGFβ-1, -2, -3.

Methods and protocols using BMPs and mimetics thereof are well-known in the art. In preferred embodiments, fibroblasts are transdifferentiated to cardiac muscle cells. Such methods and protocols are described in the art by Wang et al 2014, Cao et al 2016, Zhang et al., 2016.

The term "fibroblast" as used herein, refers to a type of cell that synthesizes the extracellular matrix and collagen, the structural framework (stroma) for animal tissues, and that plays a critical role in wound healing. Fibroblasts are the most common cells of connective tissue in animals.

The term "cardiac muscle cell", as used herein, refers to are the muscle cells (myocytes) that make up the cardiac muscle (heart muscle). Typical marker protein for cardiac muscle cells include but are not restricted to NPPB (natriuretic peptide B), MYL7 (myosin, light chain 7), NPPA (natriuretic peptide A), MYBPC3 (myosin binding protein C, cardiac), TNNT2 (troponin T type 2 (cardiac)), BMP10 (bone morphogenetic protein 10), TNNI3 (troponin I type 3 (cardiac)), MYH6 (myosin, heavy chain 6, cardiac muscle, alpha), ANKRD1 (ankyrin repeat domain 1 (cardiac muscle)), RD3L (retinal degeneration 3-like), SBK2 (SH3 domain binding kinase family, member 2), and MYL4 (myosin, light chain 4, alkali; atrial, embryonic).

In further alternative embodiments, said method is a method to reprogram a cell and wherein (a) the cell is a fibroblast; and/or (b) the cell reprograms into a pluripotent stem cell; and/or (c) the reprogramming further comprises contacting the cell with at least one compound selected from the group of Oct-4, Sox-2, Klf-4 or c-Myc.

"Reprogramming", as used herein, includes the transformation of an initially cell, for example a fibroblast, to different type of cell, such as a pluripotent stem cell. Protocols for the reprogramming of fibroblasts to pluripotent stem cells using BMPs or BMP mimetic compounds are disclosed in the art, for example by Chen et al., Cell Res. 2011 Jan; 21(1): 205-212. The reprogramming of fibroblasts to pluripotent stem cells using a BMP mimetic compound as described herein can be done using all Yamanaka factors (Oct-4, Sox-2, Klf-4 and c-Myc). However, in preferred embodiments, the BMP mimetic compound is only used in combination with Oct-4 and Sox-2 or in combination with Oct-4. Additional references describing BMP mimetics in reprogramming of somatic cells to pluripotent cells include in the context of a) murine cells Chen et al. (Chen et al., Cell Res. 2011 Jan; 21(1): 205-212) and Samavarchi-Tehrani et al. (Samavarchi-Tehrani et al., Cell Stem Cell. 2010 Jul 2;7(1):64-77) and b) human cells Hayashi et al. (Hayashi, Y. et al., Proc Natl Acad Sci U S A. 2016 Nov 15;113(46):13057-13062), which are all herein incorporated by reference.

In a second aspect, the present invention is directed to a compound of the invention as described above for use in the treatment of osteoporosis, bone fracture, osteonecrosis, osteoarthritis, rheumatoid arthritis, spondylosis, anterior lumbar fusion or long bone fracture.

The compound for use is thus defined as described above for the compounds used in the context of the methods for differentiating, transdifferentiating or reprogramming a cell.

The term "osteoporosis", as used herein, is defined as a systemic skeletal disease characterized by low bone mass and micro-architectural deterioration of bone tissue, with a consequent increase in bone fragility and susceptibility to fracture. The clinical definition of osteoporosis is a condition in which the bone mineral density (BMD) or bone mineral concentration (BMC) is greater than about 2.5 standard deviations (SD) below the mean of young healthy women. Severe osteoporosis is defined as having a BMD or BMC greater than about 2.5 SD below the mean of young healthy women and the presence of one or more fragility fractures. Since bone loss is not strictly confined to specific sites, osteoporosis can manifest itself in various ways including alveolar, femoral, radial, vertebral or wrist bone loss or fracture incidence, postmenopausal bone loss, severely reduced bone mass, fracture incidence or rate of bone loss.

The term "bone fracture", as used herein, means one of various physical injuries of a bone, based on a complete or incomplete disruption of the continuity of a bone, which are classified according to anatomical location (epiphyseal, metaphyseal, diaphyseal, intra-articular, proximal, midshaft, distal, etc.), degree of fracture (complete, incomplete), direction of fracture (transverse, oblique, spiral, longitudinal), presence of open wound (open, closed), number of fractures (simple, linear, segmental, comminuted, etc.), stability of fracture (stable, unstable), displacement of fracture, etc.

"Osteonecrosis", as used herein, is cellular death (necrosis) of bone components due to interruption of the blood supply. Without blood, the bone tissue dies and the bone collapses. If osteonecrosis/avascular necrosis involves the bones of a joint, it often leads to destruction of the joint articular surfaces.

The term "osteoarthritis", as used herein, is a type of joint disease that results from breakdown of joint cartilage and underlying bone. The most common symptoms are joint pain and stiffness. Other symptoms may include joint swelling, decreased range of motion, and when the back is affected weakness or numbness of the arms and legs. Unlike other types of arthritis, only the joints are typically affected. Causes include previous joint injury, abnormal joint or limb development, and inherited factors.

"Rheumatoid arthritis", as used herein, is a long-term autoimmune disorder that primarily affects joints. It typically results in warm, swollen, and painful joints. Most commonly, the wrist and hands are involved, with the same joints typically involved on both sides of the body. Rheumatoid arthritis may also result in a low red blood cell count, inflammation around the lungs, and inflammation around the heart.

The term "spondylosis", as used herein, refers to a broad term meaning degeneration of the spinal column from any cause. In the more narrow sense it refers to spinal osteoarthrosis, the age-related wear and tear of the spinal column, which is the most common cause of spondylosis. The degenerative process in osteoarthritis chiefly affects the vertebral bodies, the neural foramina and the facet joints (facet syndrome). If severe, it may cause pressure on nerve roots with subsequent sensory or motor disturbances, such as pain, paresthesia, and muscle weakness in the limbs.

"Anterior lumbar fusion" or "anterior lumbar interbody fusion (ALIF)", as used interchangeably herein, is similar to the posterior lumbar interbody fusion (PLIF), except that in the ALIF, the disc space is fused by approaching the spine through the abdomen instead of through the lower back. This surgery treatment is well-known in the art.

In a third aspect, the present invention relates to a method for determining bone morphogenetic protein (BMP) mimetic activity of a compound of interest, said method comprising:
(a) culturing a pluripotent stem cell, wherein said stem cell comprises an inducible mesoderm or cardiac reporter molecule;
(b) after at least one day of step (a) and for a time frame of up to five days, contacting the cell with the compound of interest and with a BMP inhibitor, preferably said BMP inhibitor is selected from the group consisting of 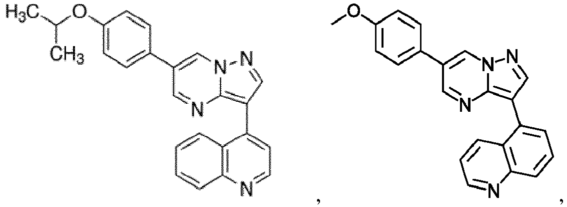 Noggin (NOG) and Chordin;
(c) after the time frame of step (b), replacing the medium of the cultured stem cell;
(d) after at least nine days of step (a), comparing the presence of the reporter molecule in the stem cell with the presence of the same reporter molecule in a control cell, wherein in step (b) the control cell is (I) only contacted with the BMP inhibitor; or (II) contacted with the BMP inhibitor and a compound having BMP mimetic activity; and
(e)
   (I) wherein the control cell has only been contacted with the BMP inhibitor and the compound of interest has BMP mimetic activity when the presence of the reporter molecule in the stem cell is significantly reduced compared to the presence of the reporter molecule in the control cell; or
   (II) wherein the control cell has been contacted with the BMP inhibitor and a compound having BMP mimetic activity and the compound of interest has BMP mimetic activity when the presence of the reporter molecule in the stem cell does not significantly differ compared to the presence of the reporter molecule in the control cell.

In various embodiments, the inducible mesoderm or cardiac reporter molecule is a nucleic acid molecule comprising a promoter of a cardiac muscle indicator gene and a reporter molecule, wherein the promoter and the reporter molecule are operatively coupled.

The term "Noggin (NOG)", as used herein, refers to a protein that is involved in the development of many body tissues, including nerve tissue, muscles, and bones. Preferably, the protein is a mammalian protein, more preferred a mouse or human protein. The reference sequence number (RefSeq) of the protein is NP_005441 (human) and NP_032737 (mouse). The according reference sequence numbers (RefSeq) of the mRNAs are NM_005450 (human) and NM_008711 (mouse).

The term "Chordin", as used herein, refers to a protein that is a bone morphogenetic protein antagonist composed of four small cysteine-rich domains. Preferably, the protein is a mammalian protein, more preferred a mouse or human protein. There are several isoforms of Chordin. The reference sequence number (RefSeq) of the human isoform 1 is NP_003732.2 (the corresponding mRNA RefSeq is NM_003741.3), the reference sequence number (RefSeq) of the human isoform 2 is NP_001291401.1 (the corresponding mRNA RefSeq is NM_001304472.1), and the reference sequence number (RefSeq) of the human isoform 3 is NP_001291402.1 (the corresponding mRNA RefSeq is NM_001304473.1). The reference sequence number (RefSeq) of the mouse isoform 1 is NP_034023.1 (the corresponding mRNA RefSeq is NM_009893.2) and the reference sequence number (RefSeq) of the human isoform 2 is NP_001264970.1 (the corresponding mRNA RefSeq is NM_001278041.1).

The term "bone morphogenetic protein (BMP) mimetic activity", as used herein, refers to molecules whose exposure to a cellular system results in the same effects as the exposure of BMPs. The mimetic effect of the compound of interest may be comparable to the molecular effects derived from the exposure of one or more of the following BMPs: BMP2/4, BMP5/6/7 /8a /8b, BMP9/10, and BMP12/13/14. Preferably, the effect of the mimetic compound mimetics the effects of BMP4, BMP2, BMP7 or BMP9. In various embodiments, the term "BMP mimetic activity" means that the exposure of the mimetic compound results in at least 50%, at least 60%, at least 65%, at least 70%, at least 75%, at least 80%, at least 85%, at least 90%, at least 95% or at least 99% of the phosphorylation of Smad1/5 compared to the exposure of one or more BMPs as mentioned-above. Preferably, the mimetic compound and the BMP are applied to the same cellular system (e.g. cell culture, animal, patient) in the same amount/concentration and for the same time period. In other preferred embodiments, the exposure of the mimetic compound to the cellular system results in at least 50%, at least 60%, at least 65%, at least 70%, at least 75%, at least 80%, at least 85%, at least 90%, at least 95% or at least 99% of Smad1/5 dimerization, Smad1/5 interaction to Smad4 or Smad1/5-Smad4 nuclear localization compared to the exposure of one or more BMPs as mentioned-above. To determine these parameters, the mimetic compound and the BMP are applied to the same cellular system in the same amount/concentration and for the same time period. Alternatively, the BMP mimetic activity may be determined by measuring the expression of BMP genes, such as BMP2/4/5/6/7 /8a /8b/9/10/12/13 or /14, or BMP targeted genes, such as Id-, Id-2, Osteocalcin, Osteopontin, or Collagen 1. In preferred embodiments, the exposure of the mimetic compound to the cellular system results in at least 50%, at least 60%, at least 65%, at least 70%, at least 75%, at least 80%, at least 85%, at least 90%, at least 95% or at least 99% of Id-1 and/or Id-2 expression compared to the exposure of one or more BMPs as mentioned-above. To compare the expression, all parameters are comparable or identical with the exception of exposure to the mimetic compound and BMPs.

The term "culturing" or "cell culturing", as used herein, refers to maintenance or growth of a mammalian cell in a (liquid) culture medium under a controlled set of physical conditions.

By "nucleic acid molecule", as used herein, is meant a molecule having nucleotides. The nucleic acid can be single, double, or multiple stranded and may comprise modified or unmodified nucleotides or non-nucleotides or various mixtures and combinations thereof. "Nucleic acid molecule" also refers to all species of DNA and RNA.

"RNA" or "ribonucleic acid", as interchangeably used herein, relates to a chain of nucleotides wherein the nucleotides contain the sugar ribose and bases selected from the group of adenine (A), cytosine (C), guanine (G), or uracil (U). "DNA" or "deoxyribonucleic acid", as interchangeably used herein, relates to a chain of nucleotides wherein the nucleotides contain the sugar 2' -deoxyribose and bases selected from adenine (A), guanine (G), cytosine (C) and thymine (T). The term "mRNA" refers to messenger RNA.

The term "base" or "nucleobase", as interchangeably used herein, relates to nitrogen-containing biological compounds (nitrogenous bases) found linked to a sugar within nucleosides - the basic building blocks of deoxyribonucleic acid (DNA) and ribonucleic acid (RNA). Their ability to form base pairs and to stack upon one another lead directly to the helical structure of DNA and RNA. The primary, or canonical, nucleobases are cytosine (DNA and RNA), guanine (DNA and RNA), adenine (DNA and RNA), thymine (DNA) and uracil (RNA), abbreviated as C, G, A, T, and U, respectively. Because A, G, C, and T appear in the DNA, these molecules are called DNA-bases; A, G, C, and U are called RNA-bases. Uracil and thymine are identical except that uracil lacks the 5' methyl group. Adenine and guanine belong to the double-ringed class of molecules called purines (abbreviated as R). Cytosine, thymine, and uracil are all pyrimidines (abbreviated as Y). Other bases that do not function as normal parts of the genetic code are termed non-canonical. Nucleobases that can be used to be coupled to the organic molecule to form a compound of the library of the present invention are thymine, cytosine, uracil, 4-acetylcytidine, 5-(carboxyhydroxymethyl)uridine, 2'-O-methylcytidine, 5-carboxymethylaminomethyl-2-thiouridine, 5-carboxymethylaminomethyluridine, dihydrouridine, 2'-O-methylpseudouridine, 1-methylpseudouridine, 3-methylcytidine, 5-methylcytidine, 5-methylaminomethyluridine, 5-methoxyaminomethyl-2-thiouridine, 5-methoxycarbonylmethyl-2-thiouridine, 5-methoxycarbonylmethyluridine, 5-methoxyuridine, uridine-5-oxyacetic acid-methylester, uridine-5-oxyacetic acid, pseudouridine, 2-thiocytidine, 5-methyl-2-thiouridine, 2-thiouridine, 4-thiouridine, 5-methyluridine, 2'-O-alkyluridine, 2'-O-alkylthynndine, 2'-O-alkylcytidine and 3-(3-amino-3-carboxy-propyl)uridine.

The term "promoter", as used herein, refers to a region of DNA upstream from the transcription start that is typically involved in binding RNA polymerase and other proteins in order to initiate transcription. Reference herein to a "promoter" is to be taken in its broadest context and includes the transcriptional regulatory sequences derived from a classical eukaryotic genomic gene, including the TATA box which is required for accurate transcription initiation, with or without a CCAAT box sequence and additional regulatory elements (i.e. upstream activating sequences, enhancers and silencers) which alter gene expression in response to developmental and/or external stimuli, or in a tissue- specific manner. Consequently, a repressible promoter's rate of transcription decreases in response to a repressing agent. An inducible promoter's rate of transcription increases in response to an inducing agent. A constitutive promoter's rate of transcription is not specifically regulated, though it can vary under the influence of general metabolic conditions. The term "promoter" also includes the transcriptional regulatory sequences of a classical prokaryotic gene, in which case it may include a -35 box sequence and/or a -10 box transcriptional regulatory sequences. The term "promoter" is also used to describe a synthetic or fusion molecule, or derivative which confers, activates or enhances expression of a nucleic acid molecule in a cell, tissue or organ. In some embodiments, the promoter is capable of being transcribed by a RNA polymerase I, RNA polymerase II, RNA polymerase III, RNA polymerase IV and/or RNA polymerase V. In some embodiments, the promoter is capable of being transcribed by a RNA polymerase III. In some embodiments, the promoter is capable of being transcribed by a RNA polymerase III only. In some embodiments, the promoter sequence comprises a nucleotide of 100-1000 nucleotides, preferably 200-700 nucleotides, more preferably 300-500 nucleotides located at the 5'-end of one of the following genes: NPPB (natriuretic peptide B), MYL7 (myosin, light chain 7), NPPA (natriuretic peptide A), MYBPC3 (myosin binding protein C, cardiac), TNNT2 (troponin T type 2 (cardiac)), BMP10 (bone morphogenetic protein 10), TNNI3 (troponin I type 3 (cardiac)), MYH6 (myosin, heavy chain 6, cardiac muscle, alpha), ANKRD1 (ankyrin repeat domain 1 (cardiac muscle)), RD3L (retinal degeneration 3-like), SBK2 (SH3 domain binding kinase family, member 2), and MYL4 (myosin, light chain 4, alkali; atrial, embryonic). The skilled person may determine the length of the promoter sequence dependent on the investigated species. Preferably, the species is mouse.

The term "operatively linked" or "operatively coupled", as interchangeably used herein, refers to the ability of the promoter to directly or indirectly promote transcription of the DNA oligonucleotide sequence of the reporter molecule into an RNA oligonucleotide molecule.

The term "cardiac muscle indicator gene", as used herein, refers to a gene whose expression is specific for cardiac muscle cells or whose expression is significantly up or down regulated in cardiac muscle cells. In preferred embodiments, the cardiac muscle indicator gene is one or more genes selected from the following group: NPPB (natriuretic peptide B), MYL7 (myosin, light chain 7), NPPA (natriuretic peptide A), MYBPC3 (myosin binding protein C, cardiac), TNNT2 (troponin T type 2 (cardiac)), BMP10 (bone morphogenetic protein 10), TNNI3 (troponin I type 3 (cardiac)), MYH6 (myosin, heavy chain 6, cardiac muscle, alpha), ANKRD1 (ankyrin repeat domain 1 (cardiac muscle)), RD3L (retinal degeneration 3-like), SBK2 (SH3 domain binding kinase family, member 2), and MYL4 (myosin, light chain 4, alkali; atrial, embryonic).

The term "reporter molecule", as used herein, refers to molecules useful for detecting the conversion of a cell. For instance, the reporter molecule may detect the qualitative or quantitative expression of a promoter of a mesodermal or cardiac muscle indicator gene. These molecules are often called detectable molecules and such phrases are used interchangeably herein. Molecules detectable by spectroscopic, photochemical, biochemical, immunochemical, electrical, radiographic and optical means are known. Optically detectable molecules include fluorescent labels, such as fluorescein and Texas Red. Detectable molecules useful in the present invention include any biologically detectable molecules, such as enzymes. These also include molecules which interact with other molecules as a means of creating a reportable event for example as some reporter molecules used in the known BRET and FRET assays which include fragmented molecular systems. Particularly preferred are those detectable molecules which are inherently fluorescent *in vivo.*

The terms "medium," "cell culture medium," and "culture medium", as used herein, refer to a solution containing nutrients that nourish growing animal, e.g., mammalian, cells. Typically, these solutions provide essential and nonessential amino acids, vitamins, energy sources, lipids, and trace elements required by the cell for minimal growth and/or survival. The solution may also contain components that enhance growth and/or survival above the minimal rate, including hormones and growth factors. The solution is preferably formulated to a pH and salt concentration optimal for cell survival and proliferation. In one embodiment, the medium is a defined medium. Defined media are media in which all components have a known chemical structure. In another embodiment of the invention, the medium may contain an amino acid(s) derived from any source or method known in the art, including, but not limited to, an amino acid(s) derived either from single amino acid addition(s) or from peptone or protein hydrolysate (including animal or plant source(s)) addition(s).

The term "comparing the presence of the reporter molecule in the stem cell with the presence of the same reporter molecule in a control cell", as used herein, refers to the comparison of the presence/signals caused by the reporter molecule. The signals may be compared qualitatively or quantitatively. The presence of the reporter molecule may be detected directly or indirectly. In preferred embodiments, the presence/signals of the reporter molecules are quantitatively detected. In more preferred embodiments, the presence/signal of the reporter molecule of the control cell is set 100% and the presence/signal of the reporter molecule of the cell contacted with the BMP mimetic compound is adapted to the value of the control experiment.

The term "significant", as used herein, relates to a specific value determined for a BMP mimetic compound of the invention in relation to a comparable value determined for a corresponding control. The determined values may be used to determine the BMP mimetic activity properties as defined herein. In various embodiments, the reference/control value may be considered to be 100%. In still further embodiments, the significant value of the BMP mimetic compound of the invention may be at least 110%, at least 120%, at least 130%, at least 140%, at least 150% or at least 200% of the reference value. In alternative embodiments, the significant value of the BMP mimetic compound of the invention may be not more than 90%, not more than 80%, not more than 70%, not more than 50%, not more than 30% or not more than 10% of the control/reference value. When the control is a positive control (a cell treated with DHM1 [compound (21)] and a compound having BMP mimetic activity), the value determined for the mimetic compound of interest does not significantly differ from the control/reference value. In preferred embodiments of this method, the value determined for the BMP mimetic compound of interest is not more than 25%, not more than 20%, not more 15%, not more than 10%, not more than 5% or not more than 2% of the value determined for the control.

In various embodiments, the compound having BMP mimetic activity and applied to the control cell is a compound selected from the group consisting of

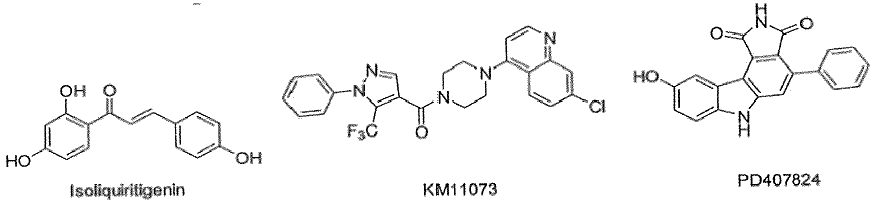

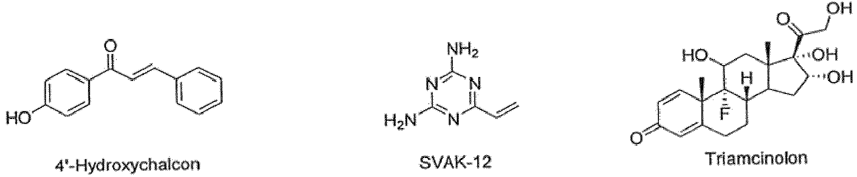

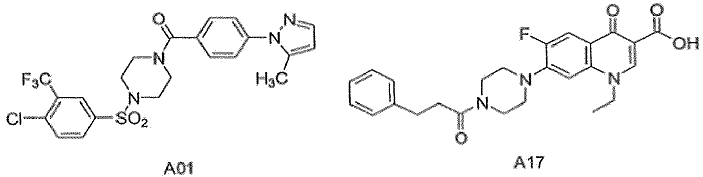

In various embodiments, the pluripotent stem cells is cultured in step (a) and/or in step (c) with differentiation medium that does not comprise the leukemia inhibitory factor (LIF).

The term "differentiation medium", as used herein, refers to a cell culture medium that can be used for the differentiation of (stem) cells. In preferred embodiments, the differentiation medium does not contain the leukemia inhibitory factor (LIF). In other preferred embodiments, the differentiation medium comprises DMEM, FBS, GlutaMAX, NEAA, Pen Strep and 2-Mercaptoethanol. "Leukemia Inhibitory Factor (LIF)", as used herein, refers to a mammalian protein, originally cloned and sequenced by Gearing et al. (1987) EMBO J. 6, 3995- 4002 which enables the derivation, growth and maintenance of undifferentiated embryonic stem cells derived from the inner cell mass of blastocysts. It refers also to splice variants of LIF and to variants of LIF wherein one or more amino acids are mutated, inserted or deleted with the restriction that the variant LIF is at least 95% identical to wild type LIF and that it enables the derivation, growth and maintenance of undifferentiated embryonic stem cells derived from the inner cell mass of blastocysts.

In various embodiments, the method is directed to methods wherein (a) the pluripotent stem cell is a murine embryonic stem cell, preferably a CGR8 cell; and/or (b) said method comprises an additional step of replacing the medium eight days after the culturing step (a); and/or (c) the compound of interest is further tested for BMP mimetic activity using a reporter construct comprising a BMP-responsive element.

The cell line CGR8 is commercially available and well-known in the art. Said cell line may be purchased from the European Collection of Authenticated Cell Cultures (ECACC) under the catalogue number ECACC 07032901.

A "reporter construct", as used herein, refers to a nucleic acid construct that can be transformed into a cell of interest, and generally comprises one or more reporter genes encoding a reporter molecule as defined herein under transcriptional control of a promoter. The one or more reporter genes of a reporter construct serve to provide a "detectable signal" upon expression. The detectable signal is any measurable parameter which evidences, in a qualitative or quantitative way, the expression of the reporter gene product (reporter molecule) in the host. Examples of detectable signals of reporter genes suitable for use in the presently disclosed embodiments include protein fluorescence (e.g., the fluorescence emission of green fluorescent protein (GFP), red fluorescent protein (REP), or yellow fluorescent protein (YFP) and enzyme activity (e.g., [3-galactosidase activity), which are well known in the art. Further suitable detectable signals include, but are not limited to, the turbidity, light scattering, or optical density of a cell suspension (indicative of cell growth resulting from reporter activity).

The term "BMP-responsive element", as used herein, refers to a nucleic sequence that can bind BMP, preferably BMP4, and subsequently increases the expression of a gene of interest. Such BMP-responsive elements can be found, for example, in the promoter sequence of Id-1 and are well-known in the art. Katagiri et al. (Katagiri, T. et al. Genes Cells. 2002 Sep;7(9):949-60) and Korchynskyi et al. (Korchynskyi O, ten Dijke P. J Biol Chem. 2002 Feb 15;277(7):4883-91. Epub 2001 Nov 29. 10.1074/jbc.M111023200) describe such element.

In various embodiments of the present invention, the promoter of the cardiac muscle indicator gene is the promoter of one of the following genes: NPPB (natriuretic peptide B), MYL7 (myosin, light chain 7), NPPA (natriuretic peptide A), MYBPC3 (myosin binding protein C, cardiac), TNNT2 (troponin T type 2 (cardiac)), BMP10 (bone morphogenetic protein 10), TNNI3 (troponin I type 3 (cardiac)), MYH6 (myosin, heavy chain 6, cardiac muscle, alpha), ANKRD1 (ankyrin repeat domain 1 (cardiac muscle)), RD3L (retinal degeneration 3-like), SBK2 (SH3 domain binding kinase family, member 2), and MYL4 (myosin, light chain 4, alkali; atrial, embryonic), preferably MYH6.

In various embodiments, the reporter molecule is a fluorescence reporter molecule, preferably green fluorescent protein (GFP).

The term "fluorescence reporter molecule", as used herein, generally refers to any kind of molecule which can be visualized by means of fluorescence detection. In preferred embodiments, the fluorescence reporter molecule is a fluorescence reporter protein. Preferably, the fluorescence reporter protein of the present invention may be a green fluorescence protein (GFP) from *Aequorea victoria* including any known functional analogues thereof in which the amino acid sequence of wild type GFP has been altered by amino acid deletion, addition, or substitution. Suitable GFP analogues for use in the present invention include, but are not limited to, EGFP (enhanced green fluorescence protein), EYFP (enhanced yellow fluorescence protein), and ECFP (enhanced cyan fluorescence protein). These fluorescence reporter proteins are well known in the art, and their nucleotide sequence can, e.g., be commercially purchased from Clontech™, USA. Other fluorescence reporter proteins of the present invention include, but are not limited to, *Aequorea coerulescence* jellyfish fluorescent protein (AcGPF1), DsRed, HcRed, AsRed, ZsGreen, ZsYellow, AmCyan fluorescent protein (BD Clontech, USA), Renilla GFP (Stratagene, USA), monomeric Kusabira-Orange (mKO1 and mKO2) and monomeric Azami-Green (mAG) (MBL Medical & Biological Laboratories Co., Ltd.), and any functional fragment or derivative thereof.

In various embodiments, the contacting step (b) is done at least two days, preferably three days after the culturing step (a) and/or the time frame of the contacting step (b) is five days, four days, three days, two days, or, preferably, one day.

In various embodiments of the present invention, the comparing step (d) is done eleven days after the culturing step (a).

"Plurality" or "more than one", as used herein, is defined as two or more than two, in particular 2, 3, 4, 5, 6, 7, 8, 9, 10 or more, more preferably 100 or more, 500 or more, 1000 or more, 1500 or more, 3000 or more, 5000 or more, 10000 or more or 50000 or more. In various embodiments, the library of the present invention comprises at least ten molecules, preferably at least 50 different molecules, more preferably at least 100 different molecules.

"At least one", as used herein, relates to one or more, in particular 1, 2, 3, 4, 5, 6, 7, 8, 9, 10 or more.

The term "protein", as used herein, relates to one or more associated polypeptides, wherein the polypeptides consist of amino acids coupled by peptide (amide) bonds. The term polypeptide refers to a polymeric compound comprised of covalently linked amino acid residues. The amino acids are preferably the 20 naturally occurring amino acids glycine, alanine, valine, leucine, isoleucine, phenylalanine, cysteine, methionine, proline, serine, threonine, glutamine, asparagine, aspartic acid, glutamic acid, histidine, lysine, arginine, tyrosine and tryptophan.

The term "contacting", as used herein in the context of mimetic compounds of interest and cells, refers generally to providing access of one component, reagent, analyte or sample to the cell. For example, contacting can involve mixing a solution comprising a BMP mimetic compound and a cell. The solution comprising the mimetic compound may also comprise another component or reagent, such as dimethyl sulfoxide (DMSO) or a detergent, which facilitates mixing, interaction, uptake, or other physical or chemical phenomenon advantageous to the contact between components, reagents, analytes and/or samples.

The term "sequence", as used herein, relates to the primary nucleotide sequence of nucleic acid molecules or the primary amino acid sequence of a protein.

Methods required to put the present invention into practise, such as cloning protocols, detection of fluorescence intensity or enzymatic activity are well-known in the art.

### EXAMPLES

### Methods

### Chemicals and agents

DAPI (Carl Roth)
DMH-1 (Selleck Chemicals)
DMSO (Agros Organics)
Formaldehyde (Carl Roth)
Gelatin solution 0,1 % (PAN Biotech)

**Media**

| | | | |
|---|---|---|---|
| Cultivation medium: | 87% | DMEM | Thermo Fisher |
| | 10% | FBS | PAN Biotech |
| | 1% | GlutaMAX | Thermo Fisher |
| | 1% | NEAA | Thermo Fisher |
| | 1% | Pen Strep | Thermo Fisher |
| | 0,1 mM | 2-Mercaptoethanol | Thermo Fisher |
| | 15 ng/mL | LIF | |
| | | | |
| Differentiation medium (1): | 87% | DMEM | Thermo Fisher |
| (D0, D4, D8) | 10% | FBS | PAN Biotech |
| | 1% | GlutaMAX | Thermo Fisher |
| | 1% | NEAA | Thermo Fisher |
| | 1% | Pen Strep | Thermo Fisher |
| | 0,1 mM | 2-Mercaptoethanol | Thermo Fisher |
| | | | |
| Differentiation medium (2): | 93% | DMEM | Thermo Fisher |
| (D3) | 4% | FBS | PAN Biotech |
| | 1% | GlutaMAX | Thermo Fisher |
| | 1% | NEAA | Thermo Fisher |
| | 1% | Pen Strep | Thermo Fisher |
| | 0,1 mM | 2-Mercaptoethanol | Thermo Fisher |

**Buffer**

| | | | |
|---|---|---|---|
| Phosphate buffer (1x): | 137 mM | NaCl | Carl Roth |
| | 2,7 mM | KCl | Carl Roth |
| | 10 mM | Na₂HPO₄ | Carl Roth |
| | 1,8 mM | KH₂PO₄ | Carl Roth |

**Devices**

| | |
|---|---|
| Countess cell counter | Invitrogen |
| Echo® Liquid Handler | Labcyte |
| EVOS XL Core Mikroskop | Life Technologies |
| ImageXpress Micro XL | Molecular Devices |
| Inkubator Hera Cell 150 | Thermo Fisher |
| Matrix pipette | Thermo Fisher |
| Microplate Washer ELx405 Select CW | BioTek |
| Multidrop Combi | Thermo Scientific |

**Cell line**

| Cell line | Reporter | Organism | Publication |
|---|---|---|---|
| CGR8 | *α-Myh6-eGFP* | Mouse | Circulation 2003;107:1912-16 |

### Cell cultivation

Work with the pluripotent stem cells was performed under aseptic conditions in a sterile bench. All materials that came into contact with the cells were previously autoclaved or disinfected. The cells were cultured in an incubator at 37 ° C and 5% CO₂.

### Thawing of pluripotent stem cells

The cells which were cryopreserved at -150 ° C were carefully transferred to cultivation medium after rapid thawing in a water bath and then centrifuged (25 ° C, 3 min, 1200 rpm). The supernatant was then removed and the cells were plated out at a concentration of 20,000 cells per mL.

### Cultivation of pluripotent stem cells

The cells were passed every two to three days to a confluence of about 60-80%. For this purpose, the surface on which the cells were to be further cultivated was first coated with 0.1% gelatin solution and incubated at RT. After the cultivation medium was removed from the cells, the cells were first washed with buffer solution before they were incubated with trypsin-EDTA solution for 5 min at 37 ° C. The cells were then singulated and incubated at 37 ° C for 2 min. The reaction was then stopped by addition of cultivation medium. The cell trypsin suspension was transferred to a centrifuge tube and centrifuged (25 ° C, 3 min, 1200 rpm). The supernatant was removed and the cell pellet was resuspended in cultivation medium. The cell count was determined and a cell suspension of the desired cell concentration was prepared in a centrifuge tube. After the gelatin solution was removed, the cells were plated on the new culture surface and further cultured in the incubator (37 ° C, 5% CO₂).

### Freezing of pluripotent stem cells

In order to cryopreserve the cells, the cell pellet was resuspended in the freezing medium during the respective cell passage. For each freezing tube, the cell concentration was adjusted to 1×10⁶ cells/mL and the freezing tubes were first cooled step by step (1 ° C/min) in a freezing container to -80 ° C before they were stored the next day at -150 ° C.

### Cell count

The cell count was determined either with an automatic cell counter or with the aid of the Neubauer counting chamber. For this purpose, 10 µl of cell suspension was mixed with 10 µl of trypan blue solution and 10 µl of this suspension was added to a counting chamber and counted.

### BMP mimetics screening assay

To investigate new activators of the BMP signaling pathway, a stem cell-based high-content assay was developed using murine embryonic stem cells of the CGR8 cell line which had been stably transfected with the promoter-reporter construct α-MHC-GFP. At day 0 of the assay, 500 pluripotent stem cells were seeded in a 25 µL serum-containing medium (10% FBS) on a previously gelatin-coated 384-well cultivation plate and cultured for eleven days (37 ° C, 5% CO₂). On day 3, libraries of low molecular weight substances were transferred to the cells with the aid of the Echo® Liquid Handler, and serum-containing medium (4% FBS) was then added to each well which additionally contained the selective BMP inhibitor DHM1 at a concentration of 0.5 µM. After 24 h, on day 4, the medium was aspirated and replaced by 75 µL of serum-containing medium (10% FBS) per well. This step was repeated on day 8. On day 11, the cells were first subjected to formaldehyde fixation (16% formaldehyde solution) and DAPI stained (1: 250), and after three washings with PBS fluorescence microscopy was measured in the channels FITC (= GFP) and DAPI. The analysis was then carried out using MetaXpress 5.3 using multiwavelength cell scoring.

### Example 1: Establishing a high content screening assay to screen for BMP mimetic compounds

Figures 1-3 summarize the finding of the present inventors that a BMP-dependent time window during mESC differentiation can be identified. Especially the differentiation profile of the pro-cardiogenic effect of the unselective BMP inhibitor dorsomorphine (DM) versus the selective analogue DMH1 during the mESC differentiation provided important information to identify a highly BMP-dependent time window (Fig. 1). Between days 3-5 of the differentiation, both DM and DMH1 are cardiogenic, whereas between day 4-6, only DM is cardiogenic, which may be explained by its pronounced inhibition of TGFβ It is also shown that the selective cardiomyogenesis induced by DMH1 can be antagonized by BMP4 in a dose-dependent manner (Fig. 2). The low molecular weight compound isoliquiritigenin (Vrijens et al. 2013), which is a BMP4 mimetic compound in the absence of BMPs, also shows a dose-dependent effect. However, the effect of isoliquiritigenin is weak and said agent is a chalcone and thus pleiotropic in its biological activity.

Figure 3 summarizes the observed profile of the three most important signal pathways, which can be observed in a time-dependent manner and independent of each other during the differentiation of mESCs.

### Example 2: High content screening of BMP mimetic compounds

Based on the above-described data, a screening for low-molecular-weight BMP mimetics was carried out at a time frame of days 3-4 after the start of stem cell cultivation/differentiation. In parallel, cardiomyogenesis was stimulated with DMH1 (0.5 µM) in the same time frame (days 3-4) and the cardiomyogenesis inhibitory effect was quantified using high-content imaging in the 384-well format. Since the present screening assay uses a "negative readout", the probability of identifying false-positive hits that only affect the viability of the cells (or are toxic) is significant. Thus, the primary readout on cardiomyogenesis was combined with a cell count (DAPI staining). Subsequently, false positive hits and artefacts were eliminated by means of a visual control.

This workflow was carried out with the commercially available LOPAC substance library (obtained from Sigma-Aldrich) of pharmacologically active drugs. A total of 1408 substances were screened in the new test system (Figure 4). Substances with an activity > 50% inhibition (= 57 hits) were tested for wrong-positive activity by toxicity and artefacts (= 33 hits). For these 33 hits, a dose-dependent BMP mimetic effect should be detected so that IC₅₀ values were determined over a dose range of 5-0.01 µM. The remaining 33 hits were also checked visually for artefacts. 10 hits demonstrate such a dose-dependent effect and have thus been validated as new BMP mimetic compounds.

In further validation experiments, for three of these 10 hits, significant selectivity for the BMP signaling pathway could be demonstrated against other closely related TGFβ family signaling pathways on ligands as well as on the canonical Wnt signaling pathway. Particularly, the substances CGS-15943 and Tyrphostin AG808 demonstrate an attractive profile (Fig. 5). It should be pointed out that all 10 validated hits from the LOPAC screen (Fig. 4) are effective as new BMP mimetics in the stem cell context of mesodermal differentiation.

All documents cited herein, are hereby incorporated by reference in their entirety.

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. In addition, where features or aspects of the invention are described in terms of Markush groups, those skilled in the art will recognize that the invention is also thereby described in terms of any individual member or subgroup of members of the Markush group. Further embodiments of the invention will become apparent from the following claims.

## Claims

1. A method for differentiating, transdifferentiating or reprogramming a cell, said method comprising:
contacting said cell with at least one compound selected from the group consisting of
I) A-B-C,
wherein A, B and C are independently from each other substituted or unsubstituted 4-6-membered heterocarbocycles,
said compound comprises in total 2-6 nitrogen atoms,
said compound comprises in total at least three, preferably at least four double bonds,
wherein the heterocarbocycles A and B share two atoms, and
wherein the heterocarbocycles B and C share two atoms;
II) A-B,
wherein A and B are independently from each other substituted or unsubstituted 4-6-membered heterocarbocycles,
said compound comprises in total 2-6 nitrogen atoms,
said compound comprises in total at least two, preferably at least three double bonds, and
wherein the heterocarbocycles A and B share two atoms;
III) B-C,
wherein B and C are independently from each other substituted or unsubstituted 4-6-membered heterocarbocycles,
said compound comprises in total 2-6 nitrogen atoms,
said compound comprises in total at least two, preferably at least three double bonds, and
wherein the heterocarbocycles B and C share two atoms; 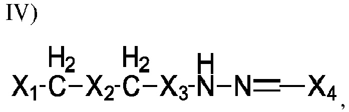
wherein
X₁ is a substituted or unsubstituted 5-6-membered aromatic (hetero)cyclyl,
X₂ is a linear substituted or unsubstituted heteroalkyl having up to 10 atoms comprising at least one nitrogen atom or a substituted or unsubstituted heterocyclyl having 4-10 atoms comprising at least one nitrogen atom;
X₃ is selected from the group consisting of C=O, S=O, O=S=O, and 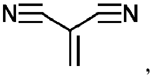 and
X₄ is a substituted or unsubstituted 4-6-membered aromatic (hetero)cyclyl; 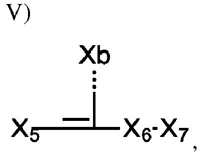
wherein
X₅ is selected from the group consisting of 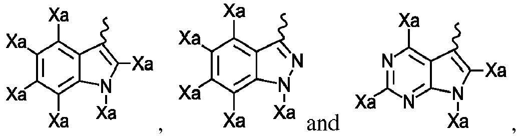 wherein Xa is H, halogen or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms,
X₆ is selected from the group consisting of C=O, S=O, O=S=O, and 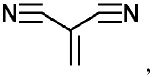 and
X₇ is a substituted or unsubstituted 5-6-membered aromatic (hetero)cyclyl,
X_{b} is selected from the group consisting of N, NO₂, COOH, COX, COX_{H}, and CONH₂, wherein
X is H or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms,
X_{H} is selected from the group consisting of F, Cl, Br and I,
------ is a) a triple bond if X_{b} is N or b) a single bond if X_{b} is NO₂, COOH, COX, COX_{H}, or CONH₂;
VI) **Yₐ-X₈-X₉-Yₐ,**
wherein
X₈ is a linear substituted or unsubstituted alkyl having up to 10 carbon atoms,
X₉ is a substituted or unsubstituted 4-6-membered (hetero)cyclyl, and
Ya is 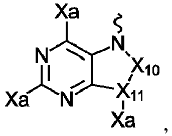 wherein
Xa is as defined above,
X₁₀ is selected from the group consisting of C=O, S=O, O=S=O, 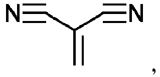 and a carbon atom linked to two moieties selected from H, halogen, a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, and substituted or unsubstituted heteroalkylaryl, each having up to 20 carbon atoms, and
X₁₁ is NH, S or O; 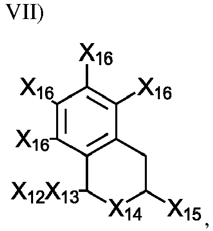
wherein
X₁₂ is NH₂, OH or SH,
X₁₃ is a linear substituted or unsubstituted alkyl having up to 10 carbon atoms,
X₁₄ is O, S or NH,
X₁₅ is a substituted or unsubstituted (hetero)cyclyl having up to 20 carbon atoms, and
X₁₆ is H or OH; 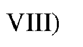 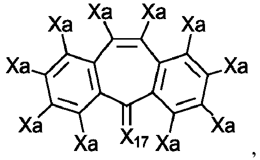
wherein
Xa is as defined above, and
X₁₇ is a substituted or unsubstituted 4-6-membered (hetero)cyclyl; 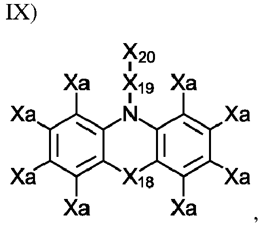
wherein
Xa is as defined above,
X₁₈ is S, O or NH,
X₁₉ is a linear substituted or unsubstituted alkyl having up to 10 carbon atoms, and
X₂₀ is a substituted or unsubstituted 4-6-membered (hetero)cyclyl; 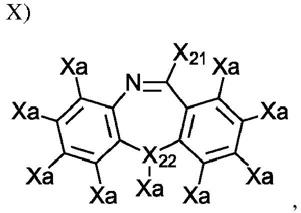
wherein
Xa is as defined above,
X₂₁ is a substituted or unsubstituted 4-6-membered (hetero)cyclyl, and
X₂₂ is S, O or NH; 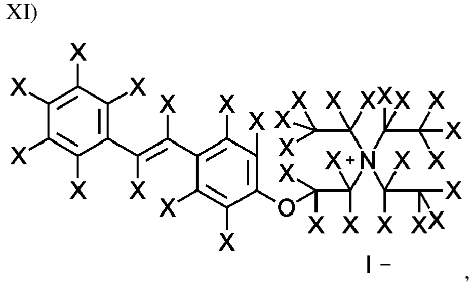
wherein
X is H or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms; and I⁻ is an anion; or 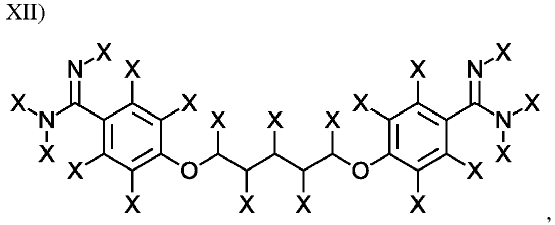 wherein
X is as defined above,
to differentiate, transdifferentiate or reprogram said cell.

2. The method according to claim 1, wherein
a) in A-B-C
i) A is a substituted or unsubstituted 6-membered heterocarbocycle;
ii) B is a substituted or unsubstituted 6-membered heterocarbocycle;
iii) C is a substituted or unsubstituted 5-membered heterocarbocycle;
iv) the number nitrogen atoms is four;
v) A comprises 1-4 halogen atoms;
vi) B comprises at least one nitrogen atom;
vii) C comprises a (hetero)aryl substituent; and/or
viii) the ring fusion between B and C is in an angle between 115°-125°; or
b) in A-B
i) A is a substituted or unsubstituted 6-membered heterocarbocycle;
ii) B is a substituted or unsubstituted 6-membered heterocarbocycle;
iii) the number nitrogen atoms is two;
iv) A comprises 1-4 halogen atoms; and/or
v) B comprises at least one nitrogen atom;
c) in B-C
i) B is a substituted or unsubstituted 6-membered heterocarbocycle;
ii) C is a substituted or unsubstituted 5-membered heterocarbocycle;
iii) the number nitrogen atoms is four;
v) B comprises at least one nitrogen atom; and/or
vi) C comprises a (hetero)aryl substituent.

3. The according to claim 1 or 2, wherein
A-B-C, A-B or B-C are selected from the group consisting of 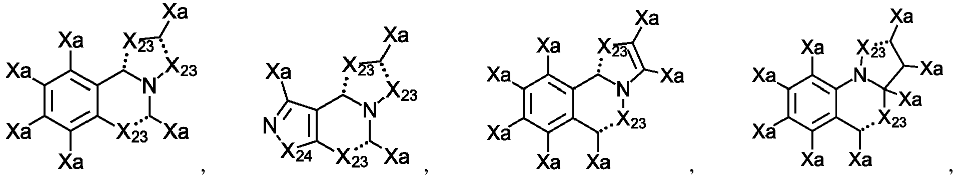 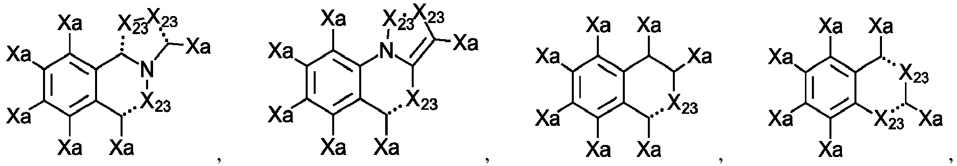 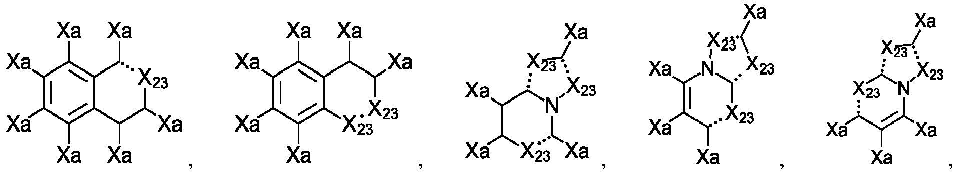 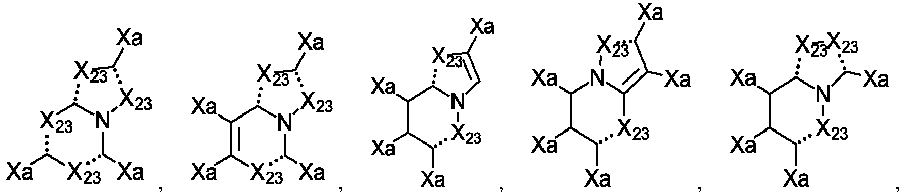 and 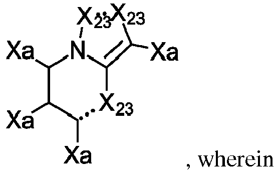
X₂₃ is N, O, S, or N-Xa,
X₂₄ is N, O, or S,
Xa is as defined above, and
------ is a) a single bond if X₂₃ is O, S, or N-Xa or b) a double bond if X₂₃ is N.

4. The method according to any of claims 1-3, wherein the compound is selected from the group consisting of 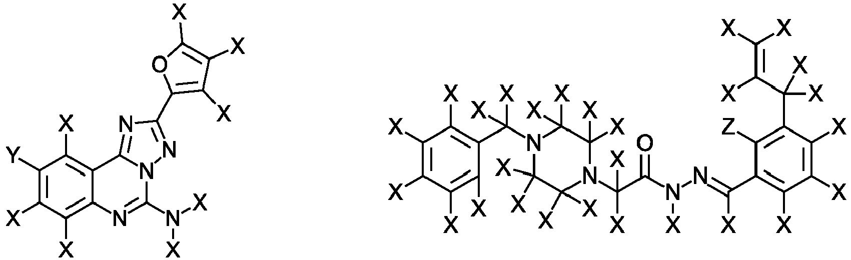 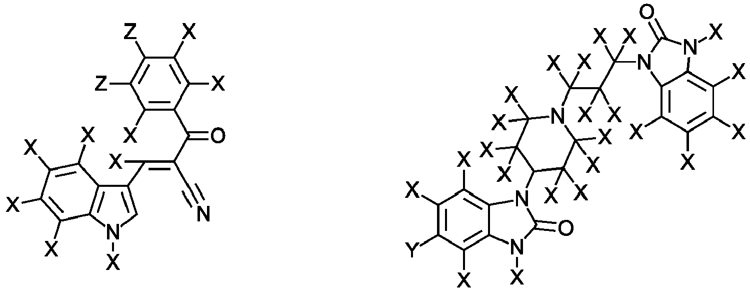 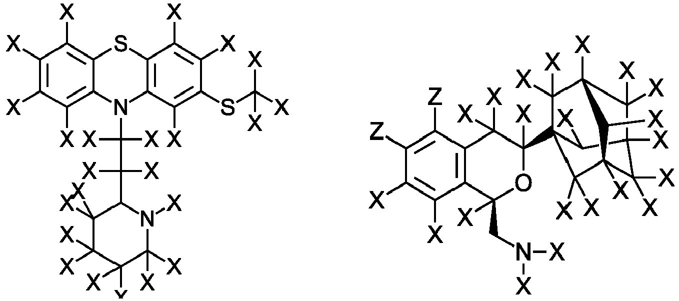 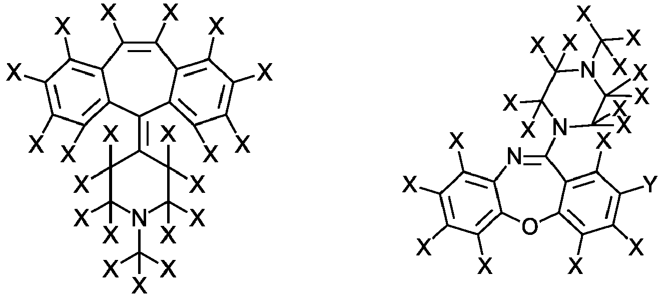 wherein
X is H or selected from the group consisting of a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl,
substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms;
Y is halogen; and
Z is a linear or branched, substituted or unsubstituted hydroxyl group having up to 10 carbon atoms.

5. The method according to claim 4, wherein X is H.

6. The method according to claims 1-5, wherein the at least one compound is selected from the group consisting of

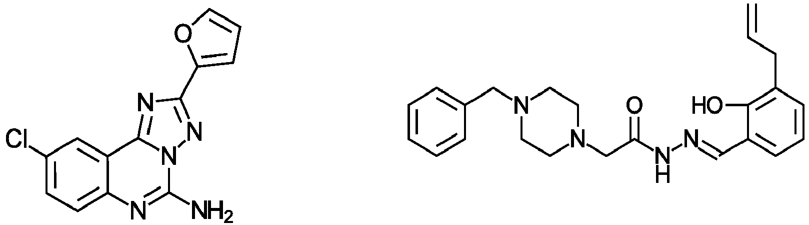

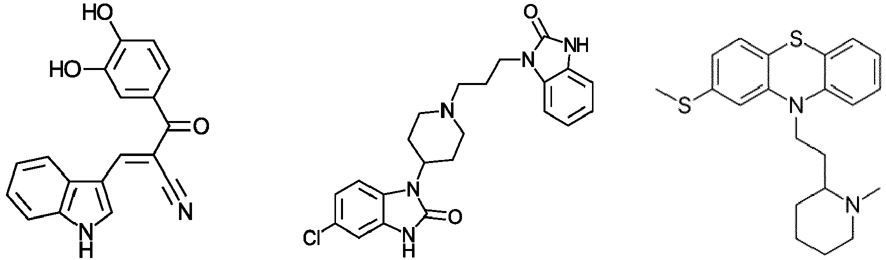

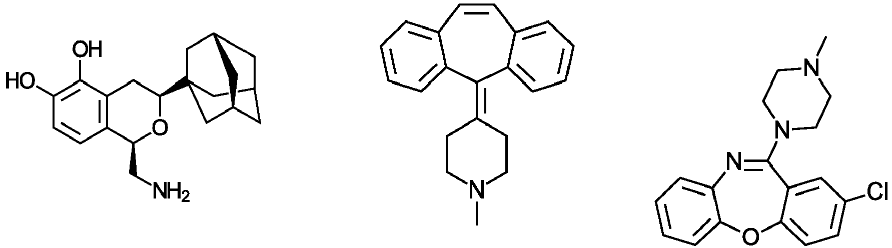

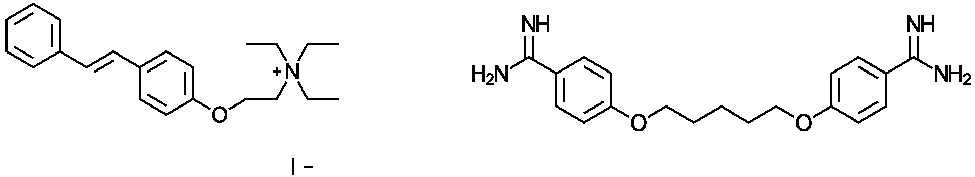

7. The method according to any one of claims 1-6, wherein
(I) said method is a method to differentiate a cell and wherein
(a) the cell is a pluripotent stem cell; and/or
(b) the cell differentiates into a multipotent cell, preferably a cardiac progenitor cell, myoblast, hematopoietic stem cell or mesenchymal stem cell; and/or
(c) the differentiation further comprises contacting the cell with at least one compound supporting differentiation;
(II) said method is a method to transdifferentiate a cell and wherein
(a) the cell is a fibroblast; and/or
(b) the cell transdifferentiates into a cardiac muscle cell; and/or
(c) the transdifferentiation further comprises contacting the cell with at least one compound supporting transdifferentiation; or
(III) said method is a method to reprogram a cell and wherein
(a) the cell is a fibroblast; and/or
(b) the cell reprograms into a pluripotent stem cell; and/or
(c) the reprogramming further comprises contacting the cell with at least one compound selected from the group of Oct-4, Sox-2, Klf-4 or c-Myc.

8. Compound according to any of claims 1-6 for use in the treatment of osteoporosis, bone fracture, osteonecrosis, osteoarthritis, rheumatoid arthritis, spondylosis, anterior lumbar fusion or long bone fracture.

9. A method for determining bone morphogenetic protein (BMP) mimetic activity of a compound of interest, said method comprising:
(a) culturing a pluripotent stem cell, wherein said stem cell comprises an inducible mesoderm or cardiac reporter molecule;
(b) after at least one day of step (a) and for a time frame of up to five days, contacting the cell with the compound of interest and with a BMP inhibitor, preferably said BMP inhibitor is selected from the group consisting of 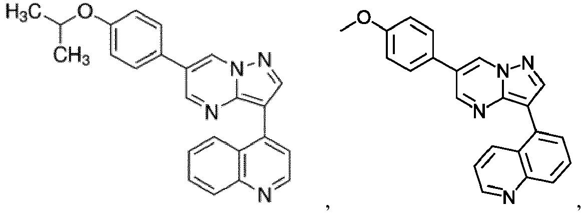 Noggin (NOG) and Chordin;
(c) after the time frame of step (b), replacing the medium of the cultured stem cell;
(d) after at least nine days of step (a), comparing the presence of the reporter molecule in the stem cell with the presence of the same reporter molecule in a control cell, wherein in step (b) the control cell is (I) only contacted with the BMP inhibitor; or (II) contacted with the BMP inhibitor and a compound having BMP mimetic activity; and
(e)
(I) wherein the control cell has only been contacted with the BMP inhibitor and the compound of interest has BMP mimetic activity when the presence of the reporter molecule in the stem cell is significantly reduced compared to the presence of the reporter molecule in the control cell; or
(II) wherein the control cell has been contacted with the BMP inhibitor and a compound having BMP mimetic activity and the compound of interest has BMP mimetic activity when the presence of the reporter molecule in the stem cell does not significantly differ compared to the presence of the reporter molecule in the control cell.

10. The method of claim 9, wherein the inducible mesoderm or cardiac reporter molecule is a nucleic acid molecule comprising a promoter of a cardiac muscle indicator gene and a reporter molecule, wherein the promoter and the reporter molecule are operatively coupled.

11. The method of claim 9(11), wherein
(a) the compound having BMP mimetic activity and applied to the control cell is a compound selected from the group consisting of 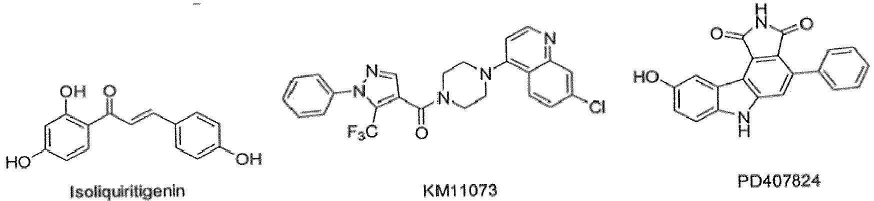 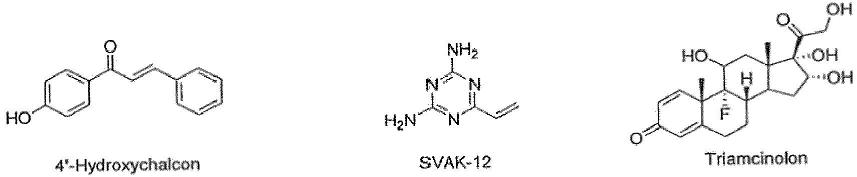 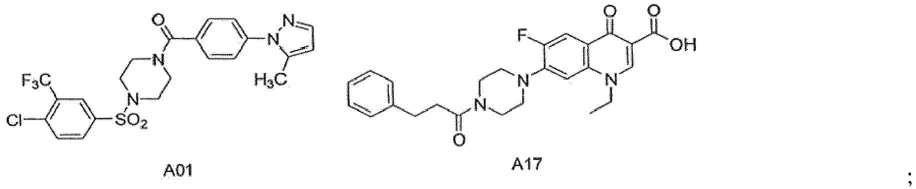 and/or
(b) the pluripotent stem cells is cultured in step (a) and/or in step (c) with differentiation medium that does not comprise the leukemia inhibitory factor (LIF).

12. The method of any one of claims 9-11, wherein
(a) the pluripotent stem cell is a murine embryonic stem cell, preferably a CGR8 cell; and/or
(b) said method comprises an additional step of replacing the medium eight days after the culturing step (a);
(c) the compound of interest is further tested for BMP mimetic activity using a reporter construct comprising a BMP-responsive element; and/or
(d) wherein the reporter molecule is a fluorescence reporter molecule, preferably green fluorescent protein (GFP).

13. The method of any one of claims 9-12, wherein the promoter of the cardiac muscle indicator gene is the promoter of one of the following genes: NPPB (natriuretic peptide B), MYL7 (myosin, light chain 7), NPPA (natriuretic peptide A), MYBPC3 (myosin binding protein C, cardiac), TNNT2 (troponin T type 2 (cardiac)), BMP10 (bone morphogenetic protein 10), TNNI3 (troponin I type 3 (cardiac)), MYH6 (myosin, heavy chain 6, cardiac muscle, alpha), ANKRD1 (ankyrin repeat domain 1 (cardiac muscle)), RD3L (retinal degeneration 3-like), SBK2 (SH3 domain binding kinase family, member 2), and MYL4 (myosin, light chain 4, alkali; atrial, embryonic), Nkx2.5, Mef2c, Isl-1, cKit, Sca1, preferably MYH6.

14. The method of any of claims 9-13, wherein the contacting step (b) is done at least two days, preferably three days after the culturing step (a) and/or the time frame of the contacting step (b) is three days, preferably one day.

15. The method of any one of claims 9-14, wherein the comparing step (d) is done eleven days after the culturing step (a).
